# EUROPEAN PATENT SPECIFICATION

(11) **EP 1 646 625 B1**
(45) Date of publication and mention of the grant of the patent: **02.07.2008**
(21) Application number: 04749151.9
(22) Date of filing: 08.07.2004
(51) Int. Cl.: C07D 403/06, C07D 207/36, A61K 31/4439, A61K 31/4025, A61P 3/10, A61P 25/28

(54) **PYRROLE-2, 5-DITHIONE DERIVATIVES AS LIVER X RECEPTOR MODULATORS**
PYRROL-2,5-DITHIONDERIVATE ALS MODULATOREN DES LIVER-X-REZEPTORS
DERIVES DE PYRROLE-2, 5-DITHIONE UTILISES COMME MODULATEURS DU RECEPTEUR X HEPATIQUE

(30) Priority: 11.07.2003 GB 0316237
(43) Date of publication of application: 19.04.2006
(73) Proprietor: AstraZeneca AB, 151 85 Södertälje (SE)
(72) Inventor: HOLM, Patrik, FIN-21600 Pargas (SE)
(86) International application number: PCT/SE2004/001115
(87) International publication number: WO 2005/005416

(56) References cited:
- WO-A1-01/03705
- WO-A2-00/21927

## Description

### Field of the invention

The present invention relates to certain novel, substituted 5-thioxo-1,5-dihydro-2*H*-pyrrol-2-one and 1*H*-pyrrole-2,5-dithione derivatives, to processes for preparing such compounds, to their the utility in modulation of nuclear hormone receptors Liver X Receptor (LXR) α (NR1H3) and/or β (NR1H2) and in treating clinical conditions including cardiovascular diseases such as atherosclerosis; inflammatory diseases, Alzheimer's disease, lipid disorders (dyslipidemias) whether or not associated with insulin resistance, type 2 diabetes and other manifestations of the metabolic syndrome, to methods for their therapeutic use and to pharmaceutical compositions containing them.

### Background of the invention

Abnormalities of cholesterol and fatty acid homeostasis, that are reflected as diverse dyslipidemias, are causal of atherosclerosis and consequently cardiovascular disease (CVD). This disease is one of the major health problems in industrialized countries and is reaching the same prevalence in adults in developing nations. Most studies show that statins reduce low density lipoproteins (LDL) cholesterol by 25-30% and the relative risk of coronary events by approximately 30%. While this beneficial effect is significant, effectively 70% of the treated cohort remains with unchanged risk. This has prompted intense research in order to identify other common abnormalities of lipid metabolism that if efficiently treated could improve the results of current CVD therapy.

The nuclear hormone receptors LXR α and β use oxysterols as natural ligands. They appear to act as cholesterol sensors with target genes that are required for cholesterol efflux from macrophages, like ATP binding casette transporter A1 (ABCA1) and apoE as well as gene products, like cholesterol ester transferase protein (CETP) and phospholipid transport protein (PLTP), that are required for the function of high density lipoprotein (HDL) in the reverse cholesterol transport. In addition, LXR upregulates lipoprotein lipase in liver and macrophages, a function that may stimulate fatty acid uptake and very low density macrophages, a function that may stimulate fatty acid uptake and very low density lipoprotein (VLDL) remodeling. In the liver, LXR ligands seem to stimulate the hepatobiliary secretion of cholesterol, a pathway controlled by the ABCG5 and ABCG8. The same cholesterol transporters appear to reduce cholesterol absorption in enterocytes, therefore influencing total body cholesterol balance. These effects of LXR stimulation could explain its remarkable anti-atherosclerotic properties observed in animal models.

Recently the synthetic LXR ligands GW3965 (Glaxo) and T-0901317 (Tularik) were reported to increase glucose tolerance in fat fed obese mouse, which was interpreted to result from reduced hepatic gluconeogenesis and increased glucose uptake in adipocytes Lafitte BA et al. (Proc Natl Acad Sci USA. 2003 Apr 29;100(9):5419-24). Activation of LXR's improves glucose tolerance through coordinated regulation of glucose metabolism in liver and adipose tissue.

WO00/21927 discloses pyrrole-2,5-diones, which are GSK-3 inhibitors and claimed to be useful in the treatment of dementias such as Alzheimer's disease, manic depression and diabetes. There is no suggestion that these compounds have activity as LXR modulators.

WO 01/03705 discloses LXR agonists and methods of using such agonists for raising HDL cholesterol levels.

The term "LXR modulator" as used herein, means a small molecule that modulates the biological activities of LXRα and/or LXRβ. More specifically, such an LXR modulator either enhances or inhibits the biological activities of LXR. If such a modulator partially or completely enhances the biological activities of LXR, it is a partial or full LXR agonist, respectively. It is the object of the present invention to provide LXR modulators. Another object of this invention is to provide LXR modulator compounds being LXR agonists.

### Description of the invention

According to a first aspect of the present invention there is provided a compound of formula I: 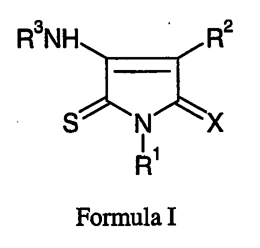 wherein:
**R¹** is selected from phenyl(1-4C)alkyl wherein the phenyl is optionally substituted by (1-4C)alkoxycarbonyl or a group of formula NR^{a}R^{b} in which R^{a} and R^{b} independently represent H or (1-4C)alkyl; heteroaryl(1-4C)alkyl wherein the heteroaryl is optionally substituted by (1-4C)alkyl or a group of formula NR^{a}R^{b} in which R^{a} and R^{b} independently represent H or (1-4C)alkyl; or a (1-6C)alkyl group which is optionally substituted by one or more of the following: fluoro, (1-4C)alkoxycarbonyl, (1-3C)alkylthio or (1-3C)alkoxy optionally substituted by one or more fluoro;
**R²** is phenyl;
**R³** is selected from phenyl, indolyl or benzofuranyl each optionally substituted by one or more of the following: (1-3C)alkanoyl, (1-4C)alkoxy optionally substituted by one or more fluoro; (1-3C)alkylthio; or a group of formula NR^{a}R^{b} in which R^{a} and R^{b} independently represent H, (1-3C)alkyl or (1-3C)alkanoyl or R^{a} and R^{b} together with the nitrogen atom to which they are attached represent morpholino;
**X** is O or S,
or a pharmaceutically acceptable salt or solvate thereof, or a solvate of such a salt.

The term heteroaryl means pyridyl, furyl or isoxazolyl each of which is optionally substituted by one or more of the following: (1-4C)alkyl or a group of formula NR^{a}R^{b} in which R^{a} and R^{b} independently represent H or (1-4C)alkyl.

Further values of **R¹, R², R³** and **X** in compounds of formula I now follow. It will be understood that such values may be used where appropriate with any of the definitions, claims or embodiments defined hereinbefore or hereinafter.

In a first group of compounds of formula I, **X** is O.

In a second group of compounds of formula I, **X** is S.

In a third group of compounds of formula I
**R¹** is selected from methyl, ethyl, propyl, butyl, 2,2,2-trifluoroethyl, benzyl, 2-methoxyethyl, 3-pyridylmethyl, 4-pyridylmethyl or 6-amino-3-pyridylmethyl;
**R²** is phenyl;
**R³** is selected from 4-methoxyphenyl, 4-difluoromethoxyphenyl or 4-morpholinophenyl;
**X** is O or S.

In a fourth group of compounds of formula I
**R¹** is selected from methyl, ethyl, 2,2,2-trifluoroethyl, benzyl, 3-pyridylmethyl or 6-amino-3-pyridylmethyl;
**R²** is phenyl;
**R³** is selected from 4-methoxyphenyl, 4-difluoromethoxyphenyl or 4-morpholinophenyl;
**X** is O or S.

In a fifth group of compounds of formula I
**R¹** is selected from ethyl, 2,2,2-trifluoroethyl, benzyl, 3-pyridylmethyl, 6-amino-3-pyridylmethyl;
**R²** is phenyl;
**R³** is selected from 4-methoxyphenyl, 4-difluoromethoxyphenyl or 4-morpholinophenyl;
**X** is O or S.

In a sixth group of compounds of formula I
**R¹** is selected from methyl, ethyl, 2,2,2-trifluoroethyl, 2-pyridylmethyl, 3-pyridylmethyl, 4-pyridylmethyl;
**R²** is phenyl;
**R³** is selected from 4-methoxyphenyl;
**X** is O or S.

In a seventh group of compounds of formula I
**R¹** is selected from 2-methoxyethyl or 6-amino-3-pyridylmethyl;
**R²** is phenyl;
**R³** is selected from 4-methoxyphenyl or 4-difluoromethoxyphenyl;
**X** is O or S.

The compounds of formula I have activity as medicaments. In particular the compounds of formula I are LXR agonists.

Specifically the present invention provides a compound selected from:
1-(2-Methoxyethyl)-4-[(4-methoxyphenyl)amino]-3-phenyl-5-thioxo-1,5-dihydro-2H-pyrrol-2-one;
1-(2-Methoxyethyl)-3-[(4-methoxyphenyl)amino]-4-phenyl-1H-pyrrole-2,5-dithione;
4-[(4-Methoxyphenyl)amino]-3-phenyl-1-(pyridin-3-ylmethyl)-5-thioxo-1,5-dihydro-2H-pyrrol-2-one;
3-[(4-Methoxyphenyl)amino]-4-phenyl-1-(pyridin-3-ylmethyl)-1H-pyrrole-2,5-dithione;
4-[(4-Methoxyphenyl)amino]-3-phenyl-1-(pyridin-4-ylmethyl)-5-thioxo-1,5-dihydro-2H-pyrrol-2-one;
3-[(4-Methoxyphenyl)amino]-4-phenyl-1-(pyridin-4-ylmethyl)-1H-pyrrole-2,5-dithione;
1-Butyl-4-[(4-methoxyphenyl)amino]-3-phenyl-5-thioxo-1,5-dihydro-2H-pyrrol-2-one;
1-Butyl-3-[(4-methoxyphenyl)amino]-4-phenyl-1H-pyrrole-2,5-dithione;
4-[(4-Methoxyphenyl)amino]-3-phenyl-5-thioxo-1-(2,2,2-trifluoroethyl)-1,5-dihydro-2H-pyrrol-2-one;
3-[(4-Methoxyphenyl)amino]-4-phenyl-1-(2,2,2-trifluoroethyl)-1H-pyrrole-2,5-dithione;
1-Benzyl-4-[(4-methoxyphenyl)amino]-3-phenyl-5-thioxo-1,5-dihydro-2H-pyrrol-2-one;
1-Benzyl-3-[(4-methoxyphenyl)amino]-4-phenyl-1H-pyrrole-2,5-dithione;
4-[(4-Methoxyphenyl)amino]-1-methyl-3-phenyl-5-thioxo-1,5-dihydro-2H-pyrrol-2-one
3-[(4-Methoxyphenyl)amino]-1-methyl-4-phenyl-1*H*-pyrrole-2,5-dithione;
1-Ethyl-4-[(4-methoxyphenyl)amino]-3-phenyl-5-thioxo-1,5-dihydro-2H-pyrrol-2-one;
1-Ethyl-3-[(4-methoxyphenyl)amino]-4-phenyl-1H-pyrrole-2,5-dithione;
1-[(6-Aminopyridin-3-yl)methyl]-4-{[4-(difluoromethoxy)phenyl]amino}-3-phenyl-5-thioxo-1,5-dihydro-2*H*-pyrrol-2-one;
1-[(6-Aminopyridin-3-yl)methyl]-3-{[4-(difluoromethoxy)phenyl]amino}-4-phenyl-1*H-*pyrrole-2,5-dithione;
1-[(6-Aminopyridin-3-yl)methyl]-4-[(4-morpholin-4-ylphenyl)amino]-3-phenyl-5-thioxo-1,5-dihydro-2*H*-pyrrol-2-one and
1-[(6-Aminopyridin-3-yl)methyl]-3-[(4-morpholin-4-ylphenyl)amino]-4-phenyl-1*H-*pyrrole-2,5-dithione;
and a pharmaceutically acceptable salt or solvate thereof, or a solvate of such a salt.

Certain compounds of the present invention may exist as tautomers. It is to be understood that the present invention encompasses all such tautomers.

### Methods of preparation

The compounds of the invention may be prepared as outlined below. However, the invention is not limited to these methods. The compounds may also be prepared as described for structurally related compounds in the prior art. The reactions can be carried out according to standard procedures or as described in the experimental section.

Compounds of formula I may be prepared by reacting a compound of formula II 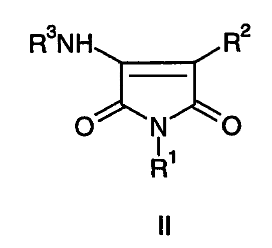 in which R¹, R² and R³ are as previously defined with a sulphurating agent, for example Lawesson's reagent, optionally in the presence of an inert organic liquid for example an aromatic hydrocarbon, e.g. toluene, at a temperature in the range of 0°C to 200°C. Compounds of formula I in which X is O may be prepared using an approximately molar equivalent of the sulphurating agent. Compounds of formula I in which X is S may be prepared using approximately two molar equivalents of the sulphurating agent.

Compounds of formula II may be prepared by reacting a compound of formula III 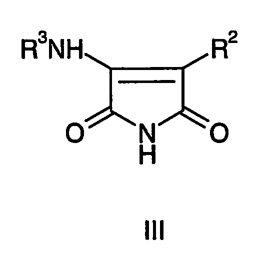 in which R² and R³ are as previously defined with a compound of formula IV

R¹OH IV

in which R¹ is as previously defined in the presence of a dialkyl azodicarboxylate, for example diethyl azodicarboxylate, and a phosphine, for example triphenylphosphine, optionally in the presence of an inert organic liquid for example an ether e.g. tetrahydrofuran at a temperature in the range of 0°C to 200°C.

Compounds of formula II may also be prepared by reacting a compound of formula V 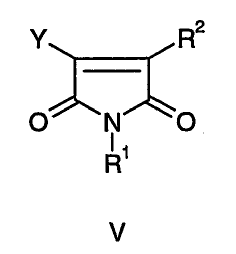 in which R¹ and R² are as previously defined and Y is a leaving group for example halo e.g. Cl, Br or I with a compound of formula VI

R³NH₂ VI

in which R³ is as previously defined optionally in the presence of an inert organic liquid for example dimethylformamide and optionally in the presence of a base for example potassium carbonate at a temperature in the range of 0°C to 250°C.

Compounds of formula III may be prepared by reacting a compound of formula VII 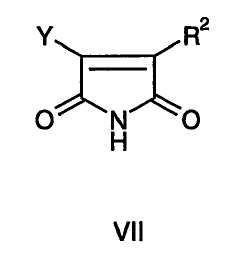 in which R² is as previously defined and Y is a leaving group for example halo eg Cl, Br or I with a compound of formula VI

R³NH₂ VI

in which R³ is as previously defined optionally in the presence of an inert organic liquid for example dimethylformamide and optionally in the presence of a base for example triethylamine at a temperature in the range of 0°C to 250°C.

Compounds of formula IV and VI are commercially available or may be prepared by methods known to those skilled in the art.

Compounds of formula V may be prepared by reacting a compound of formula VIII 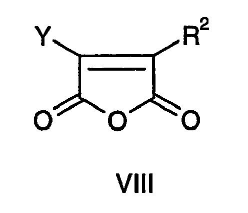 in which R² is as previously defined and Y is a leaving group for example halo eg Cl, Br or I with a compound of formula IX

R¹NH₂ IX

in which R¹ is as previously defined optionally in the presence of an organic liquid, for example glacial acetic acid at a temperature in the range of 0°C to 200°C.

Compounds of formula V may also be prepared by reacting a compound of formula VII with a compound of forumula XII

R¹L XII

in which R¹ is as previously defined and L is a leaving group for example halo eg bromo in the presence of an inert organic liquid for example dimethylformamide and optionally in the presence of a base for example potassium carbonate at a temperature in the range of -78°C to 200°C.

Compounds of formula VII may be prepared by reacting a compound of formula X 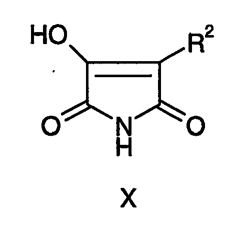 in which R² is as previously defined with a halogenating agent for example oxalyl chloride optionally in the presence of an inert organic liquid for example dichloromethane and optionally in the presence of a catalytic amount of dimethylformamide at a temperature in the range of 0°C to 200°C.

Compounds of formula VIII may be prepared by reacting a compound of formula XI 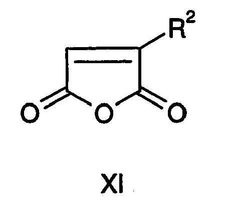 in which R² is as previously defined with a halogenating agent for example thionyl chloride optionally in the presence of an inert organic liquid for example dichloromethane and optionally in the presence of a base for example pyridine at a temperature in the range of 0°C to 200°C.

Compounds of formula IX, X, XI, and XII are commercially available or may be prepared by methods known to those skilled in the art.

Certain compounds of formula III and V are useful intermediates in the preparation of compounds of formula I and are believed to be novel and are claimed herein as useful intermediates in the preparation of compounds of formula I.

The compounds of the invention may be isolated from their reaction mixtures using conventional techniques.

Persons skilled in the art will appreciate that, in order to obtain compounds of the invention in an alternative and in some occasions, more convenient manner, the individual process steps mentioned hereinbefore may be performed in different order, and/or the individual reactions may be performed at different stage in the overall route (i.e. chemical transformations may be performed upon different intermediates to those associated hereinbefore with a particular reaction).

The expression "inert organic liquid" refers to a liquid that does not react with the starting materials, reagents, intermediates or products in a manner that adversely affects the yield of the desired product.

### Pharmaceutical preparations

The compounds of the invention will normally be administered via the oral, parenteral, intravenous, intramuscular, subcutaneous or in other injectable ways, buccal, rectal, vaginal, transdermal and/or nasal route and/or via inhalation, in the form of pharmaceutical preparations comprising the active ingredient or a pharmaceutically acceptable salt or solvate thereof, or a solvate of such a salt, in a pharmaceutically acceptable dosage form. Depending upon the disorder and patient to be treated and the route of administration, the compositions may be administered at varying doses.

Suitable daily doses of the compounds of the invention in therapeutical treatment of humans are about 0.0001-100 mg/kg body weight, preferably 0.01-10 mg/kg body weight.

Oral formulations are preferred particularly tablets or capsules which may be formulated by methods known to those skilled in the art to provide doses of the active compound in the range of 0.7 mg to 700 mg for example 1 mg, 3 mg, 5 mg, 10 mg, 25 mg, 50 mg, 100 mg and 250 mg.

According to a further aspect of the invention there is thus provided a pharmaceutical formulation including any of the compounds of the invention, or pharmaceutically acceptable derivatives thereof, in admixture with pharmaceutically acceptable adjuvants, diluents and/or carriers.

### Pharmacological properties

The compounds of formula I are useful for normalization of cholesterol homeostasis, decreasing intestinal cholesterol absorption, improving reverse cholesterol transport, improving HDL functionality, increasing HDL-cholesterol levels, decreasing LDL-cholesterol levels, decreasing cholesterol content of apoB-containing lipoproteins, stimulating cholesterol efflux from vascular cells and/or decreasing the inflammatory response of vascular cells. As a consequence of these properties the compounds of formula I are expected to have anti-atherosclerotic effects.

The compounds of formula I are useful in the prevention or treatment of cardiovascular disease in a mammal, particularly a human. The compounds of formula I are useful in the prevention or treatment of atherosclerosis in a mammal, particularly a human:
Cardiovascular disease includes but is not limited to conditions associated with atherosclerosis, arteriosclerosis, hypercholesterolemia, and other kinds of dyslipidemia that increase the risk for cardiovascular disease. In particular the compounds of formula I are useful in the treatment or prevention of cardiovascular disease, especially those involving atherosclerosis and hypercholesterolemia.

The compounds of formula I also serve to prevent lipid accumulation in, or remove lipids from, tissue deposits such as atherosclerotic plaques or xanthomas in a patient with atherosclerotic disease manifest by clinical signs such as angina, claudication, bruits, one that has suffered a mycardial infarction or transient ischemic attack, or one diagnosed by angiography, sonography or MRI.
The compounds of formula I also serve to prevent or reduce the risk of developing atherosclerosis, as well as for halting or slowing the progression of atherosclerotic disease once it has become clinically evident, comprising the administration of a prophylactically or therapeutically effective amount, as appropriate, of a compound of formula I to a mammal, including a human, who is at risk of developing atherosclerosis or who already has atherosclerotic disease.
Atherosclerosis encompasses vascular diseases and conditions that are recognized and understood by physicians practicing in the relevant fields of medicine. Atherosclerotic cardiovascular disease including restenosis following revascularization procedures, coronary heart disease (also known as coronary artery disease or ischemic heart disease), cerebrovascular disease including multi-infarct dementia, and peripheral vessel disease including erectile dysfunction are all clinical manifestations of atherosclerosis and are therefore encompassed by the terms "atherosclerosis" and "atherosclerotic disease".

The present compounds of formula I are also useful for the prophylaxis and/or treatment of clinical conditions associated with atherosclerosis such as inherent or induced hypercholesterolemia as well as inherent or induced reduced sensitivity to insulin (insulin resistance syndrome also known as metabolic syndrome) and associated metabolic disorders. These clinical conditions will include, but will not be limited to, general obesity, abdominal obesity, arterial hypertension, hyperinsulinaemia, hyperglycaemia, type 2 diabetes and the dyslipidaemia characteristically appearing with insulin resistance. This dyslipidaemia, also known as the atherogenic lipoprotein profile, is characterised by moderately elevated non-esterified fatty acids, elevated VLDL triglyceride rich particles, high Apo B levels, low HDL levels associated with low apoAI levels in the presence of small, dense, LDL particles, phenotype B.

The compounds of formula I are expected to be useful in treating patients with combined or mixed hyperlipidemias and dyslipidemias, especially low HDL levels with or without other manifestations of the metabolic syndrome.

Treatment with the compounds of formula I are expected to lower the cardiovascular morbidity and mortality associated with atherosclerosis due to their antidyslipidaemic as well as antiinflammatory properties. The cardiovascular disease conditions include macro-angiopathies of various internal organs causing myocardial infarction, congestive heart failure, cerebrovascular disease and peripheral arterial insufficiency of the lower extremities. The insulin sensitizing effect of the compounds of formula I is also expected to prevent or delay the development of type 2 diabetes from the metabolic syndrome and diabetes of pregnancy. Therefore the development of long-term complications associated with chronic hyperglycaemia in diabetes mellitus such as the micro-angiopathies causing renal disease, retinal damage and peripheral vascular disease of the lower limbs are expected to be delayed.
The compounds of formula I may also be useful for the prevention or treatment of inflammation and neurodegenerative diseases or neurological disorders. Accordingly, this invention also provides a method for preventing or treating inflammation in the CNS and a method for preventing or treating neurodegenerative diseases or disorders characterized by neuron degeneration, neuron injury or impaired plasticity or inflammation in the CNS. The neurodegenerative diseases or conditions characterized by neuron degeneration and inflammation will include but will not be limited to stroke, Alzheimer's disease, fronto-temporal dementias (taupathies), peripheral neuropathy, Parkinson's disease, dementia with Lewy bodies, Huntington's disease, amyotrophic lateral sclerosis and multiple sclerosis.
The compounds of formula I are useful in preventing or treating inflammatory conditions or diseases. These diseases or conditions will include but will not be limited to atherosclerotic diseases such as angina pectoris and myocardial infarction as well as inflammatory bowel diseases or conditions such as Crohn's disease, ulcerative colitis and distal proctitis. Compounds of formula I may also be used in other inflammatory conditions of the lung including asthma, adult respiratory distress syndrome, chronic obstructive pulmonary disease and pneumonia bronchitis.
Furthermore the compounds of formula I may be useful in treatment of various conditions outside the cardiovascular system whether or not associated with insulin resistance, like polycystic ovarian syndrome, obesity and cancer.

The present invention provides a compound of formula I to be used in method of treating and/or preventing dyslipidemias, the insulin resistance syndrome and/or metabolic disorders (as defined above) comprising the administration of a compound of formula I to a mammal (particularly a human) in need thereof.

The present invention provides a compound of formula I to be used in method of treating and/or preventing type 2 diabetes comprising the administration of an effective amount of a compound of formula I to a mammal (particularly a human) in need thereof.

The present invention provides a compound of formula I to be used in method of treating and/or preventing cardiovascular disease comprising the administration of an effective amount of a compound of formula I to a mammal (particularly a human) in need thereof.

The present invention provides a compound of formula I to be used in method of treating and/or preventing atherosclerosis comprising the administration of an effective amount of a compound of formula I to a mammal (particularly a human) in need thereof.

The present invention provides a compound of formula I to be used in method of treating and/or preventing hypercholesterolemia comprising the administration of an effective amount of a compound of formula I to a mammal (particularly a human) in need thereof.

The present invention provides a compound of formula I to be used in method of treating and/or preventing conditions associated with a need for improving reverse cholesterol transport comprising the administration of an effective amount of a compound of formula I to a mammal (particularly a human) in need thereof.

The present invention provides a compound of formula I to be used in method of treating and/or preventing conditions associated with a need for decreasing intestinal cholesterol absorption comprising the administration of an effective amount of a compound of formula I to a mammal (particularly a human) in need thereof.

The present invention provides a compound of formula I to be used in method of treating and/or preventing conditions associated with a need for increasing HDL-cholesterol levels comprising the administration of an effective amount of a compound of formula I to a mammal (particularly a human) in need thereof.

The present invention provides a compound of formula I to be used in method of treating and/or preventing conditions associated with a need for decreasing LDL-cholesterol levels comprising the administration of an effective amount of a compound of formula I to a mammal (particularly a human) in need thereof.

The present invention provides a compound of formula I to be used in method of treating and/or preventing inflammatory conditions comprising the administration of an effective amount of a compound of formula I to a mammal (particularly a human) in need thereof.

The present invention provides a compound of formula I to be used in method of treating and/or preventing Alzheimer's disease comprising the administration of an effective amount of a compound of formula I to a mammal (particularly a human) in need thereof.

The present invention provides a compound of formula I to be used in method of treating and/or preventing arteriosclerosis comprising the administration of an effective amount of a compound of formula I to a mammal (particularly a human) in need thereof.

The present invention provides a compound of formula I to be used in method of treating and/or preventing conditions associated with a need for improving HDL function comprising the administration of an effective amount of a compound of formula I to a mammal (particularly a human) in need thereof.

The present invention provides a compound of formula I to be used in method of treating and/or preventing hyperlipidemic conditions comprising the administration of an effective amount of a compound of formula I to a mammal (particularly a human) in need thereof.

In a further aspect the present invention provides the use of a compound of formula I as a medicament.

In a further aspect the present invention provides the use of a compound of formula I in the manufacture of a medicament for the treatment and/or prohylaxis of dyslipidemic conditions.

In a further aspect the present invention provides the use of a compound of formula I in the manufacture of a medicament for the treatment and/or prohylaxis of insulin resistance syndrome and/or metabolic disorders.

In a further aspect the present invention provides the use of a compound of formula I in the manufacture of a medicament for the treatment and/or prophylaxis of cardiovascular disease.

In a further aspect the present invention provides the use of a compound of formula I in the manufacture of a medicament for the treatment and/or prophylaxis of atherosclerosis.

In a further aspect the present invention provides the use of a compound of formula I in the manufacture of a medicament for the treatment and/or prophylaxis of hypercholesterolemia.

In a further aspect the present invention provides the use of a compound of formula I in the manufacture of a medicament for the treatment and/or prophylaxis of conditions associated with a need for improving reverse cholesterol transport.

In a further aspect the present invention provides the use of a compound of formula I in the manufacture of a medicament for the treatment and/or prophylaxis of conditions associated with a need for decreasing intestinal cholesterol absorption.

In a further aspect the present invention provides the use of a compound of formula I in the manufacture of a medicament for the treatment and/or prophylaxis of conditions associated with a need for increasing HDL-cholesterol levels.

In a further aspect the present invention provides the use of a compound of formula I in the manufacture of a medicament for the treatment and/or prophylaxis of conditions associated with a need for decreasing LDL-cholesterol levels.

In a further aspect the present invention provides the use of a compound of formula I in the manufacture of a medicament for the treatment and/or prophylaxis of inflammatory conditions.

In a further aspect the present invention provides the use of a compound of formula I in the manufacture of a medicament for the treatment and/or prophylaxis of Alzheimer's disease.

In a further aspect the present invention provides the use of a compound of formula I in the manufacture of a medicament for the treatment and/or prophylaxis of arteriosclerosis.

In a further aspect the present invention provides the use of a compound of formula I in the manufacture of a medicament for the treatment and/or prophylaxis of type 2 diabetes.

In a further aspect the present invention provides the use of a compound of formula I in the manufacture of a medicament for the treatment and/or prophylaxis of conditions associated with a need for improving HDL function.

In a further aspect the present invention provides the use of a compound of formula I in the manufacture of a medicament for the treatment and/or prophylaxis of hyperlipidemic conditions.

### Combination Therapy

The compounds of the invention may be combined with another therapeutic agent that is useful in the treatment of disorders associated with the development and progress of atherosclerosis such as hypertension, hyperlipidaemias, dyslipidaemias, diabetes, inflammation and obesity. The compounds of the invention may be combined with another therapeutic agent that decreases the ratio of LDL:HDL or an agent that causes a decrease in circulating levels of LDL-cholesterol. In patients with diabetes mellitus the compounds of the invention may also be combined with therapeutic agents used to treat complications related to micro-angiopathies.

In another aspect of the present invention, the compound of formula I, or a pharmaceutically acceptable salt or solvate thereof, or a solvate of such a salt, may be administered in association with cholesterol biosynthesis inhibitors, or pharmaceutically acceptable salts, solvates, solvates of such salts or prodrugs thereof. Suitable cholesterol biosynthesis inhibitors include HMG CoA reductase inhibitors, squalene synthesis inhibitors and squalene epoxidase inhibitors. A suitable squalene synthesis inhibitor is squalestatin 1 and a suitable squalene epoxidase inhibitor is NB-598.
In this aspect of the invention, the compound of formula I, or a pharmaceutically acceptable salt or solvate thereof, or a solvate of such a salt, may be administrated in association with an HMG CoA reductase inhibitor, or pharmaceutically acceptable salts, solvates, solvates of such salts or prodrugs thereof. Suitably the HMG CoA reductase inhibitor, or pharmaceutically acceptable salts, solvates, solvates of such salts or prodrugs thereof are statins well known in the art. Particular statins are selected from the group consisting of atorvastatin, fluvastatin, pitavastatin, lovastatin, mevastatin, nicostatin, nivastatin, pravastatin and simvastatin, or a pharmaceutically acceptable salt, especially sodium or calcium, solvate, solvate of such a salt or a prodrug thereof. A particular statin is atorvastatin, or a pharmaceutically acceptable salt, solvate, solvate of such a salt or a prodrug thereof. A more particular statin is atorvastatin calcium salt. A particularly preferred statin is, however, rosuvastatin, or a pharmaceutically acceptable salt, solvate, solvate of such a salt or a prodrug thereof. A preferable particular statin is rosuvastatin calcium salt.
In the present application, the term "cholesterol biosynthesis inhibitors" also includes chemical modifications of the HMG CoA reductase inhibitors, squalene synthesis inhibitors and squalene epoxidase inhibitors, such as esters, prodrugs and metabolites, whether active or inactive.

In another aspect of the present invention, the compound of formula I, or a pharmaceutically acceptable salt or solvate thereof, or a solvate of such a salt, may be administered in association with an inhibitor of the ileal bile acid transport system (IBAT inhibitor), or pharmaceutically acceptable salts, solvates, solvates of such salts or prodrugs thereof.
Suitable compounds possessing IBAT inhibitory activity have been described, see for instance the compounds described in WO 93/16055, WO 94/18183, WO 94/18184, WO 96/05188, WO 96/08484, WO 96/16051, WO 97/33882, WO 98/07449, WO 98/03818, WO 98/38182, WO 99/32478, WO 99/35135, WO 98/40375, WO 99/35153, WO 99/64409, WO 99/64410, WO 00/01687, WO 00/47568, WO 00/61568, WO 00/62810, WO 01/68906, DE 19825804, WO 00/38725, WO 00/38726, WO 00/38727, WO 00/38728, WO 00/38729, WO 01/68906, WO 01/66533, WO 02/32428, WO 02/50051, EP 864 582, EP 489 423, EP 549 967, EP 573 848, EP 624 593, EP 624 594, EP 624 595 and EP 624 596 and the contents of these patent applications are incorporated herein by reference.
Further suitable compounds possessing IBAT inhibitory activity have been described in WO 94/24087, WO 98/56757, WO 00/20392, WO 00/20393, WO 00/20410, WO 00/20437, WO 01/34570, WO 00/35889, WO 01/68637, WO 02/08211, WO 03/020710, WO 03/022825, WO 03/022830, WO 03/022286, WO 03/091232, WO 03/106482, JP 10072371, US 5070103, EP 251 315, EP 417 725, EP 869 121, EP 1 070 703 and EP 597 107 and the contents of these patent applications are incorporated herein by reference. Particular classes of IBAT inhibitors suitable for use in the present invention are benzothiazepines, and the compounds described in the claims, particularly claim 1, of WO 00/01687, WO 96/08484 and WO 97/33882 are incorporated herein by reference. Other suitable classes of IBAT inhibitors are the 1,2-benzothiazepines, 1,4-benzothiazepines and 1,5-benzothiazepines. A further suitable class of IBAT inhibitors is the 1,2,5-benzothiadiazepines.
One particular suitable compound possessing IBAT inhibitory activity is (3R,5R)-3-butyl-3-ethyl-1,1-dioxido-5-phenyl-2,3,4,5-tetrahydro-1,4-benzothiazepin-8-yl β-D-glucopyranosiduronic acid (EP 864 582). A further suitable compound possessing IBAT inhibitory activity is S-8921 (EP 597 107).
In another aspect of the present invention, the compound of formula I, or a pharmaceutically acceptable salt or solvate thereof, or a solvate of such a salt, may be administered in association with a cholesterol absorption antagonist, or pharmaceutically acceptable salts, solvates, solvates of such salts or prodrugs thereof, for example azetidinones such as ezetrol (zetia, ezetimibe) and those described in US 5,767,115 which are incorporated herein by reference. Suitable compounds possessing cholesterol absorption antagonist activity have been described, see for instance the compounds described in WO 02/50027, WO 02/66464, WO 04/005247, WO 04/000803, WO 04/000804 and WO 04/000805 which are incorporated herein by reference.
In another aspect of the present invention, the compound of formula I, or a pharmaceutically acceptable salt or solvate thereof, or a solvate of such a salt, may be administered in association with a bile acid sequestrant or pharmaceutically acceptable salts, solvates, solvates of such salts or prodrugs thereof. Suitable bile acid sequestrants include cholestyramine, cholestipol and cosevelam hydrochloride.

In another aspect of the invention, the compound of formula I, or a pharmaceutically acceptable salt or solvate thereof, or a solvate of such a salt, may be administered in association with a peroxisome proliferator-activated receptor (PPAR) modulating agent. PPAR modulating agents include but are not limited to a PPAR alpha and/or gamma and/or delta agonist, or pharmaceutically acceptable salts, solvates, solvates of such salts or prodrugs thereof. Suitable PPAR alpha and/or gamma and/or delta agonists, pharmaceutically acceptable salts, solvates, solvates of such salts or prodrugs thereof are well known in the art. These include the compounds described in WO 01/12187, WO 01/12612, WO 99/62870, WO 99/62872, WO 99/62871, WO 98/57941, WO 01/40170, WO 04/000790, WO 04/000295, WO 04/000294, WO 03/051822, WO 03/051821, WO 02/096863, WO 03/051826, WO 02/085844, WO 01/40172, J Med Chem, 1996, 39, 665, Expert Opinion on Therapeutic Patents, 10 (5), 623-634 (in particular the compounds described in the patent applications listed on page 634) and J Med Chem, 2000, 43, 527 which are all incorporated herein by reference. Particularly a PPAR alpha and/or gamma and/or delta agonist refers to muraglitazar (BMS 298585), rivoglitazone (CS-011), netoglitazone (MCC-555), balaglitazone (DRF-2593, NN-2344), clofibrate, fenofibrate, bezafibrate, gemfibrozil, ciprofibrate, pioglitazone, rosiglitazone, AVE-0847, AVE-8134, CLX-0921, DRF-10945, DRF-4832, LY-518674, LY-818, LY-929, 641597, GW-590735, GW-677954, GW-501516, MBX-102, ONO-5129, KRP-101, R-483 (BM131258), TAK-559 or TAK-654. Particularly a PPAR alpha and/or gamma and/or delta agonist refers to tesaglitazar ((S)-2-ethoxy-3-[4-(2-{4-methanesulphonyl-oxyphenyl}ethoxy)phenyl]propanoic acid) and pharmaceutically acceptable salts thereof. In yet another aspect of the invention, the compound of formula I, or a pharmaceutically acceptable salt or solvate thereof, or a solvate of such a salt, may be administered in association with a pyruvate dehydrogenase kinase (PDK) inhibitor, or a pharmaceutically acceptable salt, solvate, solvate of such a salt or a prodrug thereof, or modulators of nuclear receptors such as retenoid X receptor (RXR), or a pharmaceutically acceptable salt, solvate, solvate of such a salt or a prodrug thereof.

In another aspect of the invention, the compound of formula I, or a pharmaceutically acceptable salt or solvate thereof, or a solvate of such a salt, may be administered in association with a cholesteryl ester transfer protein (CETP) inhibitor, or pharmaceutically acceptable salts, solvates, solvates of such salts or prodrugs thereof, for example those referenced and described in WO 00/38725 page 7 line 22 - page 10, line 17 which are incorporated herein by reference.

In another aspect of the invention, the compound of formula I, or a pharmaceutically acceptable salt or solvate thereof, or a solvate of such a salt, may be administered in association with a microsomal transfer protein (MTP) inhibitor, or pharmaceutically acceptable salts, solvates, solvates of such salts or prodrugs thereof, for example implipatide and those described in WO 03/004020, WO 03/002533, WO 02/083658 and WO 00/242291, and the contents of these patent applications are incorporated herein by reference, or those described in Science, 282, 751-54, 1998 which are incorporated herein by reference.
In another aspect of the invention, the compound of formula I, or a pharmaceutically acceptable salt or solvate thereof, or a solvate of such a salt, may be administered in association with a nicotinic acid derivative, or pharmaceutically acceptable salts, solvates, solvates of such salts or prodrugs thereof, including slow release and combination products, for example, nicotinic acid (niacin), acipimox, nicofuranose, NIASPAN® and niceritrol.
In another aspect of the invention, the compound of formula I, or a pharmaceutically acceptable salt or solvate thereof, or a solvate of such a salt, may be administered in association with a acyl coenzymA: cholesterol O-acyltransferase (ACAT) inhibitor, or pharmaceutically acceptable salts, solvates, solvates of such salts or prodrugs thereof, for example CS-505, eflucimibe (F-12511) and SMP-797.
In yet another aspect of the invention, the compound of formula I, or a pharmaceutically acceptable salt or solvate thereof, or a solvate of such a salt, may be administered in association with modulators of nuclear receptors such as farnesoid X receptor (FXR), or pharmaceutically acceptable salt, solvate, solvate of such a salt or a prodrug thereof.
In another aspect of the invention, the compound of formula I, or a pharmaceutically acceptable salt or solvate thereof, or a solvate of such a salt, may be administered in association with a phytosterol compound, or pharmaceutically acceptable salts, solvates, solvates of such salts or prodrugs thereof, for example stanols.

In another aspect of the invention, the compound of formula I, or a pharmaceutically acceptable salt or solvate thereof, or a solvate of such a salt, may be administered in association with other therapies for the treatment of metabolic syndrome or type 2 diabetes and its associated complications, these include biguanide drugs, for example metformin, phenformin and buformin, insulin (synthetic insulin analogues, amylin) and oral antihyperglycemics (these are divided into prandial glucose regulators and alpha-glucosidase inhibitors). An example of an alpha-glucosidase inhibitor is acarbose or voglibose or miglitol. An example of a prandial glucose regulator is repaglinide or nateglinide.
In another aspect of the invention, the compound of formula I, or a pharmaceutically acceptable salt or solvate thereof, or a solvate of such a salt, may be administered in association with a sulfonylurea for example: glimepiride, glibenclamide (glyburide), gliclazide, glipizide, gliquidone, chloropropamide, tolbutamide, acetohexamide, glycopyramide, carbutamide, glibonuride, glisoxepid, glybuthiazole, glibuzole, glyhexamide, glymidine, glypinamide, phenbutamide, tolcylamide and tolazamide. Preferably the sulfonylurea is glimepiride or glibenclamide (glyburide). More preferably the sulfonylurea is glimepiride. Therefore the present invention includes administration of a compound of the present invention in conjunction with one, two or more existing therapies described in this paragraph. The doses of the other existing therapies for the treatment of type 2 diabetes and its associated complications will be those known in the art and approved for use by regulatory bodies for example the FDA and may be found in the Orange Book published by the FDA. Alternatively smaller doses may be used as a result of the benefits derived from the combination.

In another aspect of the invention, the compound of formula I, or a pharmaceutically acceptable salt or solvate thereof, or a solvate of such a salt, may be administered in association with an antihypertensive compound for example an angiotensin converting enzyme (ACE) inhibitor, an angiotensin II receptor antagonist, an andrenergic blocker, an alpha andrenergic blocker, a beta andrenergic blocker, a mixed alpha/beta andrenergic blocker, an andrenergic stimulant, calcium channel blocker, an AT-1 blocker, a saluretic, a diuretic or a vasodilator, or pharmaceutically acceptable salts, solvates, solvates of such salts or prodrugs thereof. Particular ACE inhibitors or pharmaceutically acceptable salts, solvates, solvate of such salts or prodrugs thereof, including active metabolites, which can be used in combination with a compound of formula I include but are not limited to, the following compounds: alacepril, alatriopril, ancovenin, benazepril, benazepril hydrochloride, benazeprilat, benzoylcaptopril, captopril, captopril-cysteine, captopril-glutathione, ceranopril, cilazapril, cilazaprilat, delapril, delapril-diacid, enalapril, enalaprilat, enapril, epicaptopril, foroxymithine, fosfenopril, fosenopril, fosenopril sodium, fosinopril, fosinopril sodium, fosinoprilat, fosinoprilic acid, hemorphin-4, imidapril, indolapril, indolaprilat, lisinopril, lyciumin A, lyciumin B, moexipril, moexiprilat, muracein A, muracein B, muracein C, pentopril, perindopril, perindoprilat, pivalopril, pivopril, quinapril, quinapril hydrochloride, quinaprilat, ramipril, ramiprilat, spirapril, spirapril hydrochloride, spiraprilat, spiropril, spiropril hydrochloride, temocapril, temocapril hydrochloride, teprotide, trandolapril, trandolaprilat, zofenopril and zofenoprilat. Preferred ACE inhibitors for use in the present invention are ramipril, ramiprilat, lisinopril, enalapril and enalaprilat. More preferred ACE inhibitors for uses in the present invention are ramipril and ramiprilat. Preferred angiotensin II receptor antagonists, pharmaceutically acceptable salts, solvates, solvate of such salts or a prodrugs thereof for use in combination with a compound of formula I include, but are not limited to, compounds: candesartan, candesartan cilexetil, losartan, valsartan, irbesartan, telmisartan and eprosartan. Particularly preferred angiotensin II receptor antagonists or pharmaceutically acceptable derivatives thereof for use in the present invention are candesartan and candesartan cilexetil.

In another aspect of the invention, the compound of formula I, or a pharmaceutically acceptable salt or solvate thereof, or a solvate of such a salt, may be administered in association with an anti-obesity compound, or pharmaceutically acceptable salts, solvates, solvates of such salts or prodrugs thereof, for example a pancreatic lipase inhibitor e.g. orlistat (EP 129,748) or an appetite (satiety) controlling substance for example sibutramine (GB 2,184,122 and US 4,929,629), a cannabinoid 1 (CB1) antagonist or inverse agonist, or pharmaceutically acceptable salts, solvates, solvates of such salts or prodrugs thereof, for example rimonabant (EP 656354 ) and as described in WO01/70700 or a melanin concentrating hormone (MCH) antagonist, or pharmaceutically acceptable salts, solvates, solvates of such salts or prodrugs thereof, for example as described in WO 04/004726.

In another aspect of the invention, the compounds of formula I, or a pharmaceutically acceptable salt or solvate thereof, or a solvate of such a salt, may be administrated in association with an anti-inflammatory agent such as glucocorticoids, non-steroidal anti-inflammatory agents (NSAID) or intestinal anti-inflammatory agents, or pharmaceutically acceptable salts, solvates, solvates of such salts or prodrugs thereof. Suitable glucocorticoids will include, but will not be limited to betametason, dexametason, methyl prednisolon, prednisolon, prednison, triamcinolon, hydrocortison, cortison and budesonid. Suitable non-steroidal anti-inflammatory agents will include, but will not be limited to indometacin, diklofenak, ibuprofen as well as acetylsalicylic acid. Suitable intestinal anti-inflammatory agents will include, but will not be limited to amino salicylates such as sulfasalazin, mesalazin, olsalazin and balsalazid.

In another aspect of the invention, the compounds of formula I, or a pharmaceutically acceptable salt or solvate thereof, or a solvate of such a salt, may be administrated in association with a cholinesterase inhibitor or an N-methyl-D-aspartate (NMDA) receptor antagonist, or pharmaceutically acceptable salts, solvates, solvates of such salts or prodrugs thereof, such as donepezil, rivastigmin or galantamin or memantin.

In an additional feature of the invention, there is provided a compound of formula I to be used in method for the treatment and/or prohylaxis of metabolic disorders in a warm-blooded animal, such as man, in need of such treatment which comprises administering to said animal an effective amount of a compound of formula I, or a pharmaceutically acceptable salt or solvate thereof, or a solvate of such a salt, in simultaneous, sequential or separate administration with an effective amount of one of the other compounds described in this combination section, or a pharmaceutically acceptable salt, solvate, solvate of such a salt or a prodrug thereof.

In an additional feature of the invention, there is provided a compound of formula I to be used in method for the treatment and/or prohylaxis of dyslipidemia in a warm-blooded animal, such as man, in need of such treatment which comprises administering to said animal an effective amount of a compound of formula I, or a pharmaceutically acceptable salt or solvate thereof, or a solvate of such a salt, in simultaneous, sequential or separate administration with an effective amount of one of the other compounds described in this combination section, or a pharmaceutically acceptable salt, solvate, solvate of such a salt or a prodrug thereof.

In an additional feature of the invention, there is provided a compound of formula I to be used in method for the treatment and/or prohylaxis of the insulin resistance syndrome in a warm-blooded animal, such as man, in need of such treatment which comprises administering to said animal an effective amount of a compound of formula I, or a pharmaceutically acceptable salt or solvate thereof, or a solvate of such a salt, in simultaneous, sequential or separate administration with an effective amount of one of the other compounds described in this combination section, or a pharmaceutically acceptable salt, solvate, solvate of such a salt or a prodrug thereof.

Therefore in an additional feature of the invention, there is provided a compound of formula I to be used in method for the treatment and/or prophylaxis of type 2 diabetes and its associated complications in a warm-blooded animal, such as man, in need of such treatment which comprises administering to said animal an effective amount of a compound of formula I, or a pharmaceutically acceptable salt or solvate thereof, or a solvate of such a salt in simultaneous, sequential or separate administration with an effective amount of one of the other compounds described in this combination section, or a pharmaceutically acceptable salt, solvate, solvate of such a salt or a prodrug thereof.

Therefore in an additional feature of the invention, there is provided a compound of formula I to be used in method of treating and/or preventing hyperlipidemic conditions in a warm-blooded animal, such as man, in need of such treatment which comprises administering to said animal an effective amount of a compound of formula I, or a pharmaceutically acceptable salt or solvate thereof, or a solvate of such a salt in simultaneous, sequential or separate administration with an effective amount of one of the other compounds described in this combination section or a pharmaceutically acceptable salt, solvate, solvate of such a salt or a prodrug thereof.

In an additional feature of the invention, there is provided a compound of formula I to be used in method for the treatment and/or prohylaxis of cardiovascular disease in a warm-blooded animal, such as man, in need of such treatment which comprises administering to said animal an effective amount of a compound of formula I, or a pharmaceutically acceptable salt or solvate thereof, or a solvate of such a salt in simultaneous, sequential or separate administration with an effective amount of one of the other compounds described in this combination section, or a pharmaceutically acceptable salt, solvate, solvate of such a salt or a prodrug thereof.

In an additional feature of the invention, there is provided a compound of formula I to be used in method for the treatment and/or prohylaxis of atherosclerosis in a warm-blooded animal, such as man, in need of such treatment which comprises administering to said animal an effective amount of a compound of formula I, or a pharmaceutically acceptable salt or solvate thereof, or a solvate of such a salt in simultaneous, sequential or separate administration with an effective amount of one of the other compounds described in this combination section, or a pharmaceutically acceptable salt, solvate, solvate of such a salt or a prodrug thereof.

In an additional feature of the invention, there is provided a compound of formula I to be used in method for the treatment and/or prohylaxis of hypercholesterolemia in a warm-blooded animal, such as man, in need of such treatment which comprises administering to said animal an effective amount of a compound of formula I, or a pharmaceutically acceptable salt or solvate thereof, or a solvate of such a salt in simultaneous, sequential or separate administration with an effective amount of one of the other compounds described in this combination section, or a pharmaceutically acceptable salt, solvate, solvate of such a salt or a prodrug thereof.

In an additional feature of the invention, there is provided a compound of formula I to be used in method for treating and/or preventing conditions associated with a need for improving reverse cholesterol transport in a warm-blooded animal, such as man, in need of such treatment which comprises administering to said animal an effective amount of a compound of formula I, or a pharmaceutically acceptable salt or solvate thereof, or a solvate of such a salt in simultaneous, sequential or separate administration with an effective amount of one of the other compounds described in this combination section, or a pharmaceutically acceptable salt, solvate, solvate of such a salt or a prodrug thereof.

In an additional feature of the invention, there is provided a compound of formula I to be used in method for treating and/or preventing conditions associated with a need for decreasing intestinal cholesterol absorption in a warm-blooded animal, such as man, in need of such treatment which comprises administering to said animal an effective amount of a compound of formula I, or a pharmaceutically acceptable salt or solvate thereof, or a solvate of such a salt in simultaneous, sequential or separate administration with an effective amount of one of the other compounds described in this combination section, or a pharmaceutically acceptable salt, solvate, solvate of such a salt or a prodrug thereof.

In an additional feature of the invention, there is provided a compound of formula I to be used in method for treating and/or preventing conditions associated with a need for increasing HDL-cholesterol levels in a warm-blooded animal, such as man, in need of such treatment which comprises administering to said animal an effective amount of a compound of formula I, or a pharmaceutically acceptable salt or solvate thereof, or a solvate of such a salt in simultaneous, sequential or separate administration with an effective amount of one of the other compounds described in this combination section, or a pharmaceutically acceptable salt, solvate, solvate of such a salt or a prodrug thereof.

In an additional feature of the invention, there is provided a compound of formula I to be used in method for treating and/or preventing conditions associated with a need for decreasing LDL-cholesterol levels in a warm-blooded animal, such as man, in need of such treatment which comprises administering to said animal an effective amount of a compound of formula I, or a pharmaceutically acceptable salt or solvate thereof, or a solvate of such a salt in simultaneous, sequential or separate administration with an effective amount of one of the other compounds described in this combination section, or a pharmaceutically acceptable salt, solvate, solvate of such a salt or a prodrug thereof.

In an additional feature of the invention, there is provided a compound of formula I to be used in method for the treatment and/or prohylaxis of inflammatory conditions in a warm-blooded animal, such as man, in need of such treatment which comprises administering to said animal an effective amount of a compound of formula I, or a pharmaceutically acceptable salt or solvate thereof, or a solvate of such a salt in simultaneous, sequential or separate administration with an effective amount of one of the other compounds described in this combination section, or a pharmaceutically acceptable salt, solvate, solvate of such a salt or a prodrug thereof.

In an additional feature of the invention, there is provided a compound of formula I to be used in method for the treatment and/or prohylaxis of Alzheimer's disease in a warm-blooded animal, such as man, in need of such treatment which comprises administering to said animal an effective amount of a compound of formula I, or a pharmaceutically acceptable salt or solvate thereof, or a solvate of such a salt in simultaneous, sequential or separate administration with an effective amount of one of the other compounds described in this combination section, or a pharmaceutically acceptable salt, solvate, solvate of such a salt or a prodrug thereof.

In an additional feature of the invention, there is provided a compound of formula I to be used in method for the treatment and/or prohylaxis of arteriosclerosis a warm-blooded animal, such as man, in need of such treatment which comprises administering to said animal an effective amount of a compound of formula I, or a pharmaceutically acceptable salt or solvate thereof, or a solvate of such a salt in simultaneous, sequential or separate administration with an effective amount of one of the other compounds described in this combination section, or a pharmaceutically acceptable salt, solvate, solvate of such a salt or a prodrug thereof.

In an additional feature of the invention, there is provided a compound of formula I to be used in method for treating and/or preventing conditions associated with a need for improving HDL function in a warm-blooded animal, such as man, in need of such treatment which comprises administering to said animal an effective amount of a compound of formula I, or a pharmaceutically acceptable salt or solvate thereof, or a solvate of such a salt in simultaneous, sequential or separate administration with an effective amount of one of the other compounds described in this combination section, or a pharmaceutically acceptable salt, solvate, solvate of such a salt or a prodrug thereof.

According to a further aspect of the invention there is provided a pharmaceutical composition which comprises a compound of formula I, or a pharmaceutically acceptable salt or solvate thereof, or a solvate of such a salt, and one of the other compounds described in this combination section or a pharmaceutically acceptable salt, solvate, solvate of such a salt or a prodrug thereof, in association with a pharmaceutically acceptable diluent or carrier.

According to a further aspect of the present invention there is provided a kit comprising a compound of formula I, or a pharmaceutically acceptable salt or solvate thereof, or a solvate of such a salt, and one of the other compounds described in this combination section or a pharmaceutically acceptable salt, solvate, solvate of such a salt or a prodrug thereof.

According to a further aspect of the present invention there is provided a kit comprising:
a) a compound of formula I, or a pharmaceutically acceptable salt or solvate thereof, or a solvate of such a salt, in a first unit dosage form;
b) one of the other compounds described in this combination section or a pharmaceutically acceptable salt, solvate, solvate of such a salt or a prodrug thereof; in a second unit dosage form; and
c) container means for containing said first and second dosage forms.

According to a further aspect of the present invention there is provided a kit comprising:
a) a compound of formula I, or a pharmaceutically acceptable salt or solvate thereof, or a solvate of such a salt, together with a pharmaceutically acceptable diluent or carrier, in a first unit dosage form;
b) one of the other compounds described in this combination section or a pharmaceutically acceptable salt, solvate, solvate of such a salt or a prodrug thereof, in a second unit dosage form; and
c) container means for containing said first and second dosage forms.

According to another feature of the invention there is provided the use of a compound of the formula I, or a pharmaceutically acceptable salt or solvate thereof, or a solvate of such a salt, and one of the other compounds described in this combination section, or a pharmaceutically acceptable salt, solvate, solvate of such a salt or a prodrug thereof, in the manufacture of a medicament for use in the treatment and/or prophylaxis of metabolic disorders in a warm-blooded animal, such as man.

According to another feature of the invention there is provided the use of a compound of the formula I, or a pharmaceutically acceptable salt or solvate thereof, or a solvate of such a salt, and one of the other compounds described in this combination section, or a pharmaceutically acceptable salt, solvate, solvate of such a salt or a prodrug thereof, in the manufacture of a medicament for use in the treatment and/or prophylaxis of dyslipidemia in a warm-blooded animal, such as man.

According to another feature of the invention there is provided the use of a compound of the formula I, or a pharmaceutically acceptable salt or solvate thereof, or a solvate of such a salt, and one of the other compounds described in this combination section, or a pharmaceutically acceptable salt, solvate, solvate of such a salt or a prodrug thereof, in the manufacture of a medicament for use in the treatment and/or prophylaxis of metabolic syndrome or type 2 diabetes and its associated complications in a warm-blooded animal, such as man.

According to another feature of the invention there is provided the use of a compound of the formula I, or a pharmaceutically acceptable salt or solvate thereof, or a solvate of such a salt, and one of the other compounds described in this combination section, or a pharmaceutically acceptable salt, solvate, solvate of such a salt or a prodrug thereof, in the manufacture of a medicament for use in the treatment and/or prophylaxis of hyperlipidaemic conditions in a warm-blooded animal, such as man.

According to another feature of the invention there is provided the use of a compound of the formula I, or a pharmaceutically acceptable salt or solvate thereof, or a solvate of such a salt, and one of the other compounds described in this combination section, or a pharmaceutically acceptable salt, solvate, solvate of such a salt or a prodrug thereof, in the manufacture of a medicament for use in the treatment and/or prophylaxis of cardiovascular disease in a warm-blooded animal, such as man.

According to another feature of the invention there is provided the use of a compound of the formula I, or a pharmaceutically acceptable salt or solvate thereof, or a solvate of such a salt, and one of the other compounds described in this combination section, or a pharmaceutically acceptable salt, solvate, solvate of such a salt or a prodrug thereof, in the manufacture of a medicament for use in the treatment and/or prophylaxis of atherosclerosis in a warm-blooded animal, such as man.

According to another feature of the invention there is provided the use of a compound of the formula I, or a pharmaceutically acceptable salt or solvate thereof, or a solvate of such a salt, and one of the other compounds described in this combination section, or a pharmaceutically acceptable salt, solvate, solvate of such a salt or a prodrug thereof, in the manufacture of a medicament for use in the treatment and/or prophylaxis of hypercholesterolemia in a warm-blooded animal, such as man.

According to another feature of the invention there is provided the use of a compound of the formula I, or a pharmaceutically acceptable salt or solvate thereof, or a solvate of such a salt, and one of the other compounds described in this combination section, or a pharmaceutically acceptable salt, solvate, solvate of such a salt or a prodrug thereof, in the manufacture of a medicament for use in the treatment and/or prophylaxis of a conditions associated with a need for improving reverse cholesterol transport in a warm-blooded animal, such as man.

According to another feature of the invention there is provided the use of a compound of the formula I, or a pharmaceutically acceptable salt or solvate thereof, or a solvate of such a salt, and one of the other compounds described in this combination section, or a pharmaceutically acceptable salt, solvate, solvate of such a salt or a prodrug thereof, in the manufacture of a medicament for use in the treatment and/or prophylaxis of a conditions associated with a need for decreasing intestinal cholesterol absorption in a warm-blooded animal, such as man.

According to another feature of the invention there is provided the use of a compound of the formula I, or a pharmaceutically acceptable salt or solvate thereof, or a solvate of such a salt, and one of the other compounds described in this combination section, or a pharmaceutically acceptable salt, solvate, solvate of such a salt or a prodrug thereof, in the manufacture of a medicament for use in the treatment and/or prophylaxis of a conditions associated with a need for increasing HDL-cholesterol levels in a warm-blooded animal, such as man.

According to another feature of the invention there is provided the use of a compound of the formula I, or a pharmaceutically acceptable salt or solvate thereof, or a solvate of such a salt, and one of the other compounds described in this combination section, or a pharmaceutically acceptable salt, solvate, solvate of such a salt or a prodrug thereof, in the manufacture of a medicament for use in the treatment and/or prophylaxis of a conditions associated with a need for decreasing LDL-cholesterol levels in a warm-blooded animal, such as man.

According to another feature of the invention there is provided the use of a compound of the formula I, or a pharmaceutically acceptable salt or solvate thereof, or a solvate of such a salt, and one of the other compounds described in this combination section, or a pharmaceutically acceptable salt, solvate, solvate of such a salt or a prodrug thereof, in the manufacture of a medicament for use in the treatment and/or prophylaxis of inflammatory conditions in a warm-blooded animal, such as man.

According to another feature of the invention there is provided the use of a compound of the formula I, or a pharmaceutically acceptable salt or solvate thereof, or a solvate of such a salt, and one of the other compounds described in this combination section, or a pharmaceutically acceptable salt, solvate, solvate of such a salt or a prodrug thereof, in the manufacture of a medicament for use in the treatment and/or prophylaxis of Alzheimer's disease in a warm-blooded animal, such as man.

According to another feature of the invention there is provided the use of a compound of the formula I, or a pharmaceutically acceptable salt or solvate thereof, or a solvate of such a salt, and one of the other compounds described in this combination section, or a pharmaceutically acceptable salt, solvate, solvate of such a salt or a prodrug thereof, in the manufacture of a medicament for use in the treatment and/or prophylaxis of arteriosclerosis in a warm-blooded animal, such as man.

According to another feature of the invention there is provided the use of a compound of the formula I, or a pharmaceutically acceptable salt or solvate thereof, or a solvate of such a salt, and one of the other compounds described in this combination section, or a pharmaceutically acceptable salt, solvate, solvate of such a salt or a prodrug thereof, in the manufacture of a medicament for use in the treatment and/or prophylaxis of a conditions associated with a need for improving HDL function in a warm-blooded animal, such as man.

According to a further aspect of the present invention there is provided a combination treatment comprising the administration of an effective amount of a compound of the formula I, or a pharmaceutically acceptable salt or solvate thereof, or a solvate of such a salt, optionally together with a pharmaceutically acceptable diluent or carrier, with the simultaneous, sequential or separate administration of an effective amount of one of the other compounds described in this combination section, or a pharmaceutically acceptable salt, solvate, solvate of such a salt or a prodrug thereof, optionally together with a pharmaceutically acceptable diluent or carrier to a warm-blooded animal, such as man in need of such therapeutic treatment.

### Examples

### Abbreviations

- DMF: *N, N*-dimethylformamide
- DMSO: dimethylsulfoxide
- EtOAc: ethyl acetate
- EtOH: ethanol
- HPLC: high performance liquid chromatography
- NMR: nuclear magnetic resonance
- THF: tetrahydrofuran
- UV: ultra violet
- h: hour
- min.: minutes
- rt: room temperature
- br: broad
- bs: broad singlet
- bt: broad triplet
- d: doublet
- dd: doublet of doublets
- m: multiplet
- q: quartet
- s: singlet
- t: triplet

### General Experimental Procedures

Flash column chromatography employed normal phase silica gel 60 (0.040-0.063 mm, Merck) or IST Isolute®SPE columns normal phase silica gel. Purifications were performed on either a Gilson preparative HPLC system with a UV triggered fraction collector, equipped with a ACE C8 5 µm 250 mm x 20 mm column, or on a Waters preparative HPLC system equipped with an ACE C8 5 µm 250 mm x 50 mm column or an ACE C8 5 µm 250 mm x 20 mm column. ¹H NMR spectra were obtained on a Varian Unity Plus, 400 MHz, operating at 9.3 T, equipped with a 5 mm switchable probe with an inner X-coil, for solutions in CDCl3 (residual CHCl₃ (δ_{H} 7.23 ppm) as internal standard), or DMSO-*d*₆ (residual DMSO (δ_{H} 2.50 ppm) as internal standard) at 300K. Chemical shifts are given in ppm. Microwave heating was performed using single node heating in a Smith Creator from Personal Chemistry, Uppsala, Sweden. Lawesson's reagent is 2,4-Bis(4-methoxyphenyl)-1,3,2,4-dithiadiphosphetane-2,4-disulfide.

### Synthesis of Starting Materials and Intermediates

### 3-Chloro-4-phenylfuran-2,5-dione

To an ice cold solution of phenylmaleic anhydride (5.74 mmol, 1.0 g) in thionyl chloride (6.0 mL) was added dropwise pyridine (11.4 mmol, 0.9 g). The reaction mixture was stirred for 60 min at 0°C, followed by heating to 75°C for 20 min. The reaction mixture was cooled to rt and the thionyl chloride was removed *in vacuo*. The crude residue was suspended in toluene (10 mL), refluxed for 10 min., followed by filtration of the hot mixture. The filtrate was concentrated to give 1.15 g (96%) of the title compound. ¹H NMR (400 MHz, CDCl₃) δ 8.05-8.00 (m, 2H), 7.59 - 7.51 (m, 3H).

### 3-Chloro-1-(2-methoxyethyl)-4-phenyl-1H-pyrrole-2,5-dione

A solution of 3-chloro-4-phenylfuran-2,5-dione (0.20 mmol, 42 mg) and 2-methoxyethylamine (0.20 mmol, 15 mg) in glacial acetic acid (1 mL) was heated in a microwave reactor at 120°C for two min.. After cooling, the solvent was evaporated at reduced pressure. The crude product was used without purification.

### 1-(2-Methoethyl)-3-[(4-methoxyphenyl)amino]-4-phenyl-1H-pyrrole-2,5-dione

3-Chloro-1-(2-methoxyethyl)-4-phenyl-1H-pyrrole-2,5-dione (0.20 mmol, 53 mg) and 4-methoxyaniline (0.48 mmol, 59 mg) were dissolved in DMF (1 mL). The mixture was heated in a microwave reactor at 150°C for five min.. After cooling, the reaction mixture was purified by HPLC (95% 0.1M ammonium acetate buffer: 5% CH₃CN → 100% CH₃CN) to give 15 mg (21%) of the title compound. ¹H NMR (400 MHz, CDCl₃) δ 7.27 (bs, 1H), 7.13-7.04 (m, 3H), 7.00-6.96 (m, 2H), 6.61-6.50 (m, 4H), 3.80 (t, J=5.6 Hz, 2H), 3.67 (s, 3H), 3.62 (t, J=5.6 Hz, 2H), 3.36 (s, 3H).

### 3-Chloro-4-phenyl-1-(pyridin-3-ylmethyl)-1H-pyrrole-2,5-dione

A solution of 3-chloro-4-phenylfuran-2,5-dione (1.00 mmol, 209 mg) and 3-(aminomethyl)pyridine (1.00 mmol, 26 mg) in glacial acetic acid (4 mL) was heated in a microwave reactor at 120°C for two min.. After cooling, the solvent was evaporated at reduced pressure. The crude product was used without purification.

### 3-[(4-Methoxyphenyl)amino]-4-phenyl-1-(pyridin-3-ylmethyl)-1H-pyrrole-2,5-dione.

To a solution of 3-chloro-4-phenyl-1-(pyridin-3-ylmethyl)-1H-pyrrole-2,5-dione (0.50 mmol, 149 mg) in DMF (1 mL) was added 4-methoxyaniline (1.10 mmol, 135 mg). The mixture was heated in a microwave reactor at 150°C for 5 min.. After cooling, the reaction mixture was purified by HPLC (95% 0.1M ammonium acetate buffer: 5% CH₃CN → 100% CH₃CN) to give 77 mg (40%) of the title compound. ¹H NMR (400 MHz, CDCl₃) δ 8.71 (d, J=1.8 Hz, 1H), 8.54 (dd, J₁=4.7 Hz, J₂=1.6 Hz, 1H), 7.80-7.75 (m, 1H), 7.35 (bs, 1H), 7.26 (dd, J₁=7.8 Hz, J₂=4.7 Hz, 1H), 7.15-7.05 (m, 3H), 6.98-6.94 (m, 2H), 6.62-6.50 (m, 4H), 4.78 (s, 2H), 3.68 (s, 3H).

### 3-[(4-Methoxyphenyl)amino]-4-phenyl-1-(pyridin-4-ylmethyl)-1H-pyrrole-2,5-dione

A mixture of 3-[(4-methoxyphenyl)amino]-4-phenyl-1H-pyrrole-2,5-dione (0.50 mmol, 147 mg), 4-hydroxymethylpyridine (0.75 mmol, 82 mg), diethyl azodicarboxylate (0.75 mmol, 131 mg) and triphenylphosphine (0.75 mmol, 197 mg) in dry THF (2 mL) was heated in a microwave reactor at 120°C for 5 min.. After cooling, the reaction mixture was purified by HPLC (95% 0.1M ammonium acetate buffer: 5% CH₃CN → 100% CH₃CN) to give 56 mg (29%) of the title compound. ¹H NMR (400 MHz, CDCl₃) δ 8.59 (bs, 2H), 7.35-7.29 (m, br, 2H), 7.25 (s, br, 1H), 7.17-7.06 (m, 3H), 7.01-6.96 (m, 2H), 6.63-6.53 (m, 4H), 4.77 (s, 2H), 3.70 (s, 3H).

### 1-Butyl-3-chloro-4-phenyl-1H-pyrrole-2,5-dione

To a solution of 3-chloro-4-phenylfuran-2,5-dione (24.0 mmol, 5.0 g) in glacial acetic acid (60 mL) was added dropwise butylamine (24.0 mmol, 1.75 g) over a period of 10 min. and the reaction mixture was refluxed for 60 min.. The reaction mixture was concentrated and partitioned between water and EtOAc and the organic layer was dried over anhydrous Na₂SO₄. After removal of the solvent, the residual oil was purified using pre-packed SiO₂ column (2x70 g) eluted with heptane (300 mL), heptane:EtOAc (95:5, 450 mL), and finally heptane:EtOAc (9:1, 450 mL) to give 2.67 g (42%) of the title compound. ¹H NMR (400 MHz, CDCl₃) δ 7.96-7.90 (m, 2H) 7.51-7.45 (m, 3H), 3.63 (t, J=7.2 Hz, 2H), 1.68-1.58 (m, 2H) 1.41-1.30 (m, 2H), 0.95 (t, J=7.3 Hz, 3H).

### 1-Butyl-3-[(4-methoxyphenyl)amino]-4-phenyl-1H-pyrrole-2,5-dione

To a solution of 1-butyl-3-chloro-4-pheny 1-1*H*-pyrrole-2,5-dione (10.1 mmol, 2.7 g) in absolute EtOH (30 mL) was added p-methoxyaniline (20.2 mmol, 2.49 g) in one portion and the mixture was refluxed for 4 h. The mixture was cooled to rt, the precipitate filtered off and washed with several portions of ice-cooled EtOH, and the solid product was finally dried over CaCl₂ to give 2.37 g (80%) of the title compound. ¹H NMR (400 MHz, CDCl3) δ 7.18 (bs, 1H), 7.13-7.05 (m, 3H), 7.01-6.96 (m, 2H), 6.62-6.52 (m, 4H), 3.69 (s, 3H), 3.61 (t, J=7.2 Hz, 2H), 1.71-1.60 (m, 2H), 1.44-1.32 (m, 2H), 0.95 (t, J=7.3 Hz, 3H).

### 3-Hydroxy-4-phenyl-1H-pyrrole-2,5-dione

Prepared according to literature procedure: C. S. Rooney, et al; J. Med. Chem., Vol. 26 (1983) pp 700-714.

### 3-Chloro-4-phenyl-1H-pyrrole-2,5-dione

To a suspension of 3-hydroxy-4-phenyl-1*H*-pyrrole-2,5-dione (25.0 g, 0.13 mol) in dichloromethane (600 mL) under an atmosphere of nitrogen was added DMF (36 mL). The suspension was cooled to ice temperature and treated with oxalyl chloride (40.0 g, 0.32 mol). The reaction mixture was subsequently refluxed overnight. After cooling to rt, silica gel was added and the reaction mixture evaporated to dryness and subjected to flash chromatography (hexane:EtOAc 80:20). Trituration with dichloromethane, filtration and drying gave 17.6 g (64%) of the title compound. ¹H NMR (400 MHz, CDCl₃) δ 7.96-7.89 (m, 2H), 7.88-7.77 (bs, 1H), 7.55-7.45 (m, 3H).

### 3-[(4-Methoxyphenyl)amino]-4-phenyl-1H-pyrrole-2,5-dione

To a solution of 3-chloro-4-phenyl-1*H*-pyrrole-2,5-dione (4.84 mmol, 1.0 g) in dry DMF (5 mL) was added 4-methoxyaniline (4.87 mmol, 600 mg) and the reaction mixture was subjected to microwave heating single node 150°C, 15 min, followed by 150°C, 10 min. The solvent was evaporated, and the crude mixture was partitioned between dichloromethane and water. The organic phase was dried over anhydrous Na₂SO4, concentrated and the residue was purified on SiO₂ (Heptane:EtOAc 3:1 → 2:1) to give 457 mg (32%) of the title compound. ¹H NMR (400 MHz, DMSO-*d*₆) δ 10.62 (s, 1H), 9.27 (s, 1H), 7.09-6.99 (m, 3H), 6.87-6.83 (m, 2H), 6.65-6.60 (m, 2H), 6.52-6.47 (m, 2H) 3.58 (s, 3H).

### 3-[(4-Methoxyphenyl)amino]-4-phenyl-1-(2,2,2-trifluoroethyl)-1H-pyrrole-2,5-dione

A solution of 3-[(4-methoxyphenyl)amino]-4-phenyl-1H-pyrrole-2,5-dione (2.11 mmol, 620 mg), diethyl azodicarboxylate (2.11 mmol, 367 mg) and triphenylphosphine (2.11 mmol, 553 mg) in dry THF (2 mL) was prepared in a sealed reaction vessel. 2,2,2-trifluoroethanol (2.11 mmol, 211 mg) was added. The mixture was stirred at 40°C for 19 h. Acetonitrile was added until some triphenylphosphine oxide was precipitated. The reaction mixture was filtered and purified by HPLC (95% 0.1M ammonium acetate buffer: 5% CH₃CN → 100% CH₃CN) to give 260 mg (33%) of the title compound. ¹H NMR (400 MHz, CDCl₃) δ 7.30 (bs, 1H), 7.18-7.06 (m, 3H), 7.01-6.96 (m, 2H), 6.64-6.52 (m, 4H), 4.23 (q, J=8.8 Hz, 2H), 3.70 (s, 3H).

### 1-Benzyl-3-[(4-methoxyphenyl)amino]-4-phenyl-1H-pyrrole-2,5-dione

A mixture of 3-[(4-methoxyphenyl)amino]-4-phenyl-1H-pyrrole-2,5-dione (0.17 mmol, 50 mg), benzylalcohol (0.17 mmol, 18 mg), diethyl azodicarboxylate (0.17 mmol, 30 mg) and triphenylphosphine (0.17 mmol, 45 mg) in dry THF (1 mL) was heated in a microwave reactor at 150°C for 6 min.. After cooling, the reaction mixture was purified by HPLC (95% 0.1M ammonium acetate buffer: 5% CH₃CN → 100% CH₃CN) to give 32 mg (49%) of the title compound. ¹H NMR (400 MHz, CDCl₃) δ 7.46-7.42 (m, 2H), 7.39-7.27 (m, 3H), 7.17 (bs, 1H), 7.14-7.05 (m, 3H), 7.00-6.96 (m, 2H), 6.61-6.51 (m, 4H), 4.77 (s, 2H), 3.69 (s, 3H

### 3-[(4-Methoxyphenyl)amino]-1-methyl-4-phenyl-1H-pyrrole-2,5-dione

A mixture of 3-[(4-methoxyphenyl)amino]-4-phenyl-1H-pyrrole-2,5-dione (0.17 mmol, 50 mg), methanol (0.17 mmol, 5 mg), diethyl azodicarboxylate (0.17 mmol, 30 mg) and triphenylphosphine (0.17 mmol, 45 mg) in dry THF (1 mL) was heated in a microwave reactor at 150°C for 6 min.. After cooling, the reaction mixture was purified by HPLC (95% 0.1M ammonium acetate buffer: 5% CH₃CN → 100% CH₃CN) to give 34 mg (65%) of the title compound. ¹H NMR (400 MHz, CDCl₃) δ 7.21 (s, br, 1H), 7.16-7.05 (m, 3H), 7.00-6.96 (m, 2H), 6.62-6.52 (m, 4H), 3.69 (s, 3H), 3.12 (s, 3H).

### 3-Chloro-1-ethyl-4-phenyl-1H-pyrrole-2,5-dione

A mixture of 3-chloro-4-phenyl-1H-pyrrole-2,5-dione (3.00 mmol, 623 mg), ethyl iodide (3.30 mmol, 515 mg) and potassium carbonate (3.30 mmol, 456 mg) in acetonitrile (10 mL) was refluxed for 3.5 h. The mixture was evaporated to dryness. The residue was taken up in ethyl acetate, washed with 1 M potassium carbonate solution and brine. Drying with sodium sulfate and evaporation under reduced pressure gave 587 mg (83%) of the desired product. ¹H NMR (400 MHz, CDCl₃) δ 7.97-7.89 (m, 2H), 7.51-7.45 (m, 3H), 3.68 (q, J=7.1 Hz, 2H), 1.25 (t, J=7.1 Hz, 3H).

### 1-Ethyl-3-[(4-methoxyphenyl)amino]-4-phenyl-1H-pyrrole-2,5-dione

3-Chloro-1-ethyl-4-phenyl-1H-pyrrole-2,5-dione (1.34 mmol, 315 mg), 4-methoxyaniline (1.47 mmol, 181 mg) and triethylamine (147 mmol, 149 mg) were dissolved in acetonitrile (4 mL). The mixture was heated in a microwave reactor at 150°C until complete reaction. After cooling, the reaction mixture was concentrated and filtrated. Purification by HPLC (95% 0.1M ammonium acetate buffer: 5% CH₃CN → 100% CH₃CN) gave 310 mg (72%) of the title compound. ¹H NMR (400 MHz, CDCl₃) δ 7.21 (bs, 1H), 7.15-7.06 (m, 3H), 7.01-6.97 (m, 2H), 6.63-6.55 (m, 4H), 3.69 (s, 3H), 3.67 (q, J=7.2 Hz, 2H), 1.27 (t, J=7.2 Hz,3H).

### tert-butyl [5-(bromomethyl)pyridin-2-yl]carbamate

Prepared according to literature procedure: WO0066557 Linschoten, M. et al, Astrazeneca AB, Nov. 9, 2000.

### tert-Butyl {5-[(3-chloro-2,5-dioxo-4-phenyl-2,5-dihydro-1H-pyrrol-1-yl)methyl]pyridin-2-yl}carbamate

3-Chloro-4-phenyl-1*H*-pyrrole-2,5-dione (1.55 g, 7.47 mmol) was dissolved in DMF (25 mL) under nitrogen atmosphere and cooled in an ice-bath. *tert*-Butyl [5-(bromomethyl)pyridin-2-yl]carbamate (2.14 g, 7.47 mmol) was added followed by anhydrous potassium carbonate (1.03 g, 7.47 mmol). The mixture was stirred for 1.5 h whereafter the cooling-bath was removed and the mixture was stirred for another two h and then neutralized with 1% HCl. Water (100 mL) was added and the mixture was extracted with CH₂Cl₂ (50 mL x 3). The extracts were combined, washed with water (100 mL x 2), dried with magnesium sulphate, filtered and evaporated. The crude product (3.41 g) was used in the next step without further purification. ¹H NMR (400 MHz, CDCl₃) δ 8.32 (d, J=2 Hz, 1H), 7.92-7.89 (m, 3H), 7.83 (bs, 1H), 7.72 (dd, J =9, 2 Hz, 1H), 7.49-7.47 (m, 3H), 4.71 (s, 2H) and 1.52 (s, 9H).

### 1-[(6-Aminopyridin-3-yl)methyl]-3-{[4-(difluoromethoxy)phenyl]amino}-4-phenyl-1H-pyrrole-2,5-dione

A mixture of *tert*-butyl {5-[(3-chloro-2,5-dioxo-4-phenyl-2,5-dihydro-1*H*-pyrrol-1-yl)methyl]pyridin-2-yl}carbamate (0.70 g, 1.7 mmol) and 4-(difluoromethoxy)aniline (0.54 g, 3.4 mmol) in DMF (4 mL) was heated in a microwave reactor at 150°C for 8 min.. The solvent was evaporated and the residue was purified on a pre-packed SiO₂column (Isolute® SI, 10g/70 mL) using CH₂Cl₂ and then CH₃OH/ CH₂Cl₂ (1:99, 2:98 and then 5:95) as eluant to give 0.4 g (54%) of the title compound. ¹H NMR (400 MHz, CDCl₃) δ 7.99 (bs, 1H), 7.67-7.62 (m, 2H), 7.14-7.04 (m, 3H), 6.91 (d, J =8 Hz, 2H), 6.78 (d, J=8 Hz, 1H), 6.72 (d, J=9 Hz, 2H), 6.63 (d, J=9 Hz, 2H), 6.33 (t, J=74 Hz, 1H) and 4.60 (s, 2H).

### 1-[(6-Aminopyridin-3-yl)methyl]-3-[(4-morpholin-4-ylphenyl)amino]-4-phenyl-1H-pyrrole-2,5-dione

A mixture of *tert*-butyl {5-[(3-chloro-2,5-dioxo-4-phenyl-2,5-dihydro-1*H*-pyrrol-1-yl)methyl]pyridin-2-yl}carbamate (0.85 g, 2.06 mmol) and 4-morpholinoaniline (0.73 g, 4.12 mmol) in DMF (4 mL) was heated in a microwave reactor at 150°C for 10 min.. Purification using preparative HPLC (C18, 50x250 mm, 60% 0.1M ammonium acetate buffer: 40% CH₃CN → 100% CH₃CN) gave 0.39 g (42%) of the title compound. ¹H NMR (400 MHz, CDCl₃) δ 8.14 (bs, 1H), 7.55 (dd, J= 8, 2 Hz, 1H), 7.28-7.23 (br, 1H), 7.13-7.04 (m, 3H), 6.95 (dd, J = 8, 2 Hz, 2H), 6.57-6.51 (m, 4H), 6.44 (d, J= 8 Hz, 1H), 4.62 (s, 2H), 4.62-4.53 (br, 2H) 3.81-3.79(m, 4H) and 3.01-2.98 (m, 4H).

### Examples

### Example 1

### 1-(2-Methoxyethyl)-4-[(4-methoxyphenyl)amino]-3-phenyl-5-thioxo-1,5-dihydro-2H-pyrrol-2-one

A mixture of 1-(2-methoxyethyl)-3-[(4-methoxyphenyl)amino]-4-phenyl-1H-pyrrole-2,5-dione (0.071 mmol, 25 mg) and Lawesson's reagent (0.071 mmol, 29 mg) in toluene (2.5 mL) was heated in a microwave reactor at 140°C for 15 min.. The solvent was evaporated at reduced pressure. The residue was redissolved in THF and purified by HPLC (95% 0.1M ammonium acetate buffer: 5% CH₃CN → 100% CH₃CN) to give 16 mg (61%) of the title compound. ¹H NMR (400 MHz, CDCl₃) δ 7.78 (bs, 1H), 7.13-6.96 (m, 5H), 6.65-6.60 (m, 2H), 6.56-6.51 (m, 2H), 4.16 (t, J=5.9, 2H), 3.72 (t, J=5.9, 2H), 3.69 (s, 3H), 3.39 (s, 3H)

### Example 2

### 1-(2-Methoxyethyl)-3-[(4-methoxyphenyl)amino]-4-phenyl-1H-pyrrole-2,5-dithione

A mixture of 1-(2-methoxyethyl)-3-[(4-methoxyphenyl)amino]-4-phenyl-1H-pyrrole-2,5-dione (0.071 mmol, 25 mg) and Lawesson's reagent (0.14 mmol, 58 mg) in toluene (2.5 mL) was heated in a microwave reactor at 180°C for 60 min.. The solvent was evaporated at reduced pressure. The residue was redissolved in THF and purified by HPLC (95% 0.1M ammonium acetate buffer: 5% CH₃CN → 100% CH₃CN) to give 14 mg (37%) of the title compound. ¹H NMR (400 MHz, CDCl₃) δ 7.41 (s, br, 1H), 7.13-6.93 (m, 5H), 6.64-6.57 (m, 2H), 6.48-6.42 (m, 2H), 4.58 (t, J=6.2, 2H), 3.75 (t, J=6.2, 2H), 3.67 (s, 3H), 3.40 (s, 3H).

### Example 3

### 4-[(4-Methoxyphenyl)amino]-3-phenyl-1-(pyridin-3-ylmethyl)-5-thioxo-1,5-dihydro-2H-pyrrol-2-one

A mixture of 3-[(4-methoxyphenyl)amino]-4-phenyl-1-(pyridin-3-ylmethyl)-1H-pyrrole-2,5-dione (0.065 mmol, 25 mg) and Lawesson's reagent (0.065 mmol, 26 mg) in toluene (2.5 mL) was heated in a microwave reactor at 140°C for 15 min.. The solvent was evaporated at reduced pressure. The residue was redissolved in THF and purified by HPLC (95% 0.1M ammonium acetate buffer: 5% CH₃CN → 100% CH₃CN) to give 19 mg (73%) of the title compound. ¹H NMR (400 MHz, CDCl₃) δ 8.76 (bs, 1H), 8.53 (d, br, 1H), 7.82-7.78 (m, 1H), 7.75 (bs, 1H), 7.28-7.23 (m, 1H), 7.14-7.03 (m, 3H), 7.00-6.95 (m, 2H), 6.63-6.59 (m, 2H), 6.55-6.50 (m, 2H), 5.13 (s, 2H), 3.68 (s, 3H).

### Example 4

### 3-[(4-Methoxyphenyl)amino]-4-phenyl-1-(pyridin-3-ylmethyl)-1H-pyrrole-2,5-dithione

A mixture of 3-[(4-methoxyphenyl)amino]-4-phenyl-1-(pyridin-3-ylmethyl)-1H-pyrrole-2,5-dione (0.065 mmol, 25 mg) and Lawesson's reagent (0.14 mmol, 58 mg) in toluene (2.5 mL) was heated in a microwave reactor at 180°C for 60 min.. The solvent was evaporated at reduced pressure. The residue was redissolved in THF and purified by HPLC (95% 0.1M ammonium acetate buffer: 5% CH₃CN → 100% CH₃CN) to give 23 mg (85%) of the title compound. ¹H NMR (400 MHz, CDCl₃) δ 8.74 (d, br, 1H), 8.53-8.50 (m, 1H), 7.81-7-76 (m, 1H), 7.41 (s, br, 1H), 7.26-7.22 (m, 1H), 7.13-7.02 (m, 3H), 6.98-6.93 (m, 2H), 6.63-6.57 (m, 2H), 6.48-6.42 (m, 2H), 5.59 (s, 2H), 3.67 (s, 3H).

### Example 5

### 4-[(4-Methoxyphenyl)amino]-3-phenyl-1-pyridin-4-ylmethyl)-5-thioxo-1,5-dihydro-2H-pyrrol-2-one

A mixture of 3-[(4-methoxyphenyl)amino]-4-phenyl-1-(pyridin-4-ylmethyl)-1H-pyrrole-2,5-dione (0.073 mmol, 28 mg) and Lawesson's reagent (0.073 mmol, 29 mg) in toluene (2.5 mL) was heated in a microwave reactor at 140°C for 15 min.. The solvent was evaporated at reduced pressure. The residue was redissolved in THF and purified by HPLC (95% 0.1M ammonium acetate buffer: 5% CH₃CN → 100% CH₃CN) to give 9 mg (31%) of the title compound. ¹H NMR (400 MHz, CDCl₃) δ 8.57 (s, br, 2H), 7.76 (s, br, 1H), 7.32 (d, br, 2H), 7.14-7.05 (m, 3H), 7.01-6.97 (m, 2H), 6.65-6.60 (m, 2H), 6.56-6.51 (m, 2H), 5.11 (s, 2H), 3.69 (s, 3H).

### Example 6

### 3-[(4-Methoxyphenyl)amino]-4-phenyl-1-pyridin-4-ylmethyl)-1H-pyrrole-2,5-dithione

A mixture of 3-[(4-methoxyphenyl)amino]-4-phenyl-1-(pyridin-4-ylmethyl)-1H-pyrrole-2,5-dione (0.073 mmol, 28 mg) and Lawesson's reagent (0.14 mmol, 59 mg) in toluene (2.5 mL) was heated in a microwave reactor at 180°C for 60 min.. The solvent was evaporated at reduced pressure. The residue was redissolved in THF and purified by HPLC (95% 0.1M ammonium acetate buffer: 5% CH₃CN → 100% CH₃CN) to give 4 mg (13%) of the title compound. ¹H NMR (400 MHz, CDCl₃) δ 8.55 (dd, J₁=6.1 Hz, J₂=4.4 Hz, 2H), 7.41 (s, br, 1H), 7.28 (dd, J₁=6.1 Hz, J₂=4.4 Hz, 2H), 7.14-7.03 (m, 3H), 7.00-6.95 (m, 2H), 6.65-6.59 (m, 2H), 6.49-6.43 (m, 2H), 5.58 (s, 2H), 3.67 (s, 3H).

### Example 7

### 1-Butyl-4-[(4-methoxyphenyl)amino]-3-phenyl-5-thioxo-1,5-dihydro-2H-pyrrol-2-one

A mixture of 1-butyl-3-[(4-methoxyphenyl)amino]-4-phenyl-1H-pyrrole-2,5-dione (0.12 mmol, 42 mg) and Lawesson's reagent (0.14 mmol, 58 mg) in toluene (1.7 mL) was heated in a microwave reactor at 120°C for five min.. The solvent was evaporated at reduced pressure. Purification by HPLC (95% 0.1M ammonium acetate buffer: 5% CH₃CN → 100% CH₃CN) gave 28 mg (64%) of the title compound. ¹H NMR (400 MHz, CDCl₃) δ 7.79 (s, br, 1H), 7.12-7.04 (m, 3H), 7.01-6.96 (m, 2H), 6.51-6.46 (m, 4H), 3.94 (t, J=7.5 Hz, 2H), 3.69 (s, 3H), 1.77-1.68 (m, 2H), 1.45-1.35 (m, 2H), 0.96 (t, J=7.3 Hz, 3H).

### Example 8

### 1-Butyl-3-[(4-methoxyphenyl)amino]-4-phenyl-1H-pyrrole-2,5-dithione

A mixture of 1-butyl-3-[(4-methoxyphenyl)amino]-4-phenyl-1H-pyrrole-2,5-dione (0.12 mmol, 42 mg) and Lawesson's reagent (0.26 mmol, 107 mg) in toluene was heated in a microwave reactor at 160°C for 25 min.. The solvent was evaporated at reduced pressure. Purification by HPLC (95% 0.1M ammonium acetate buffer: 5% CH₃CN → 100% CH₃CN) gave 28 mg (61 %) of the title compound. ¹H NMR (400 MHz, CDCl₃) δ 7.42 (s, br, 1H), 7.12-7.02 (m, 3H), 6.98-6.93 (m, 2H), 6.63-6.57 (m, 2H), 6.47-6.43 (m, 2H), 4.36-4.30 (m, 2H), 3.68 (s, 3H), 1.80-1.70 (m, 2H), 1.48-1.37 (m, 2H), 0.97 (t, J=7.7 Hz, 3H).

### Example 9

### 4-[(4-Methoxyphenyl)amino]-3-phenyl-5-thioxo-1-(2,2,2-trifluoroethyl)-1,5-dihydro-2H-pyrrol-2-one

A mixture of 3-[(4-methoxyphenyl)amino]-4-phenyl-1-(2,2,2-trifluoroethyl)-1H-pyrrole-2,5-dione (0.053 mmol, 20 mg) and Lawesson's reagent (0.053 mmol, 21 mg) in toluene (2.5 mL) was heated in a microwave reactor at 140°C for 15 min.. The solvent was evaporated at reduced pressure. The residue was redissolved in THF and purified by HPLC (95% 0.1M ammonium acetate buffer: 5% CH₃CN → 100% CH₃CN) to give 12 mg (58%) of the title compound. ¹H NMR (400 MHz, CDCl₃) δ 7.74 (s, br, 1H), 7.15-7.05 (m, 3H), 7.02-6.97 (m, 2H), 6.67-6.61 (m, 2H), 6.57-6.52 (m, 2H), 4.59 (q, J=8.6 Hz, 2H), 3.69 (s, 3H).

### Example 10

### 3-[(4-Methoxyphenyl)amino]-4-phenyl-1-(2,2m2-trifluoroethyl)-1H-pyrrole-2,5-dithione

A mixture of 3-[(4-methoxyphenyl)amino]-4-phenyl-1-(2,2,2-trifluoroethyl)-1H-pyrrole-2,5-dione (0.053 mmol, 20 mg) and Lawesson's reagent (0.11 mmol, 43 mg) in toluene (2.5 mL) was heated in a microwave reactor at 180°C for 60 min.. The solvent was evaporated at reduced pressure. The residue was redissolved in THF and purified by HPLC (95% 0.1M ammonium acetate buffer: 5% CH₃CN → 100% CH₃CN) to give 11 mg (51%) of the title compound. ¹H NMR (400 MHz, CDCl₃) δ 7.40 (s, br, 1H), 7.15-7.02 (m, 3H), 6.99-6.93 (m, 2H), 6.64-6.59 (m, 2H), 6.48-6.43 (m, 2H), 5.08 (q, J=8.4 Hz, 2H), 3.68 (s, 3H).

### Example 11

### 1-Benzyl-4-[(4-methoxyphenyl)amino]-3-phenyl-5-thioxo-1,5-dihydro-2H-pyrrol-2-one

A mixture of 1-benzyl-3-[(4-methoxyphenyl)amino]-4-phenyl-1H-pyrrole-2,5-dione (0.075 mmol, 29 mg) and Lawesson's reagent (0.075 mmol, 31 mg) in toluene (2.5 mL) was heated in a microwave reactor at 140°C for 15 min.. The solvent was evaporated at reduced pressure. The residue was redissolved in THF and purified by HPLC (95% 0.1M ammonium acetate buffer: 5% CH₃CN → 100% CH₃CN) to give 15 mg (50%) of the title compound. ¹H NMR (400 MHz, CDCl₃) δ 7.77 (s, br, 1H), 7.50-7.46 (m, 2H), 7.36-7.25 (m, 3H), 7.14-7.04 (m, 3H), 7.02-6.97 (m, 2H), 6.64-6.59 (m, 2H), 6.56-6.50 (m, 2H), 5.13 (s, 2H), 3.69 (s, 3H).

### Example 12

### 1-Benzyl-3-[(4-methoxyphenyl)amino]-4-phenyl-1H-pyrrole-2,5-dithione

A mixture of 1-benzyl-3-[(4-methoxyphenyl)amino]-4-phenyl-1H-pyrrole-2,5-dione (0.075 mmol, 29 mg) and Lawesson's reagent (0.15 mmol, 61 mg) in toluene (2.5 mL) was heated in a microwave reactor at 180°C for 60 min.. The solvent was evaporated at reduced pressure. The residue was redissolved in THF and purified by HPLC (95% 0.1M ammonium acetate buffer: 5% CH₃CN → 100% CH₃CN) to give 18 mg (57%) of the title compound. ¹H NMR (400 MHz, CDCl₃) δ 7.46-7.41 (m, 2H), 7.40 (s, br, 1H), 7.34-7.22 (m, 3H), 7.13-7.02 (m, 3H), 7.00-6.95 (m, 2H), 6.63-6.57 (m, 2H), 6.48-6.42 (m, 2H), 5.59 (s, 2H), 3.67 (s, 3H).

### Example 13

### 4-[(4-Methoxyphenyl)amino]-1-methyl-3-phenyl-5-thioxo-1,5-dihydro-2H-pyrrol-2-one

A mixture of 3-[(4-methoxyphenyl)amino]-1-methyl-4-phenyl-1H-pyrrole-2,5-dione (0.088 mmol, 27 mg) and Lawesson's reagent (0.088 mmol, 35 mg) in toluene (2.5 mL) was heated in a microwave reactor at 140°C for 15 min.. The solvent was evaporated at reduced pressure. The residue was redissolved in THF and purified by HPLC (95% 0.1M ammonium acetate buffer: 5% CH₃CN → 100% CH₃CN) to give 20 mg (70%) of the title compound. ¹H NMR (400 MHz, CDCl₃) δ 7.78 (s, br, 1H), 7.13-7.03 (m, 3H), 7.00-6.96 (m, 2H), 6.64-6.60 (m, 2H), 6.56-6.51 (m, 2H), 3.69 (s, 3H), 3.40 (s, 3H).

### Example 14

### 3-[(4-Methoxyphenyl)amino]-1-methyl-4-phenyl-1H-pyrrole-2,5-dithione

A mixture of 3-[(4-methoxyphenyl)amino]-1-methyl-4-phenyl-1H-pyrrole-2,5-dione (0.088 mmol, 27 mg) and Lawesson's reagent (0.17 mmol, 71 mg) in toluene (2.5 mL) was heated in a microwave reactor at 180°C for 60 min.. The solvent was evaporated at reduced pressure. The residue was redissolved in THF and purified by HPLC (95% 0.1M ammonium acetate buffer: 5% CH₃CN → 100% CH₃CN) to give 26 mg (87%) of the title compound. ¹H NMR (400 MHz, CDCl₃) δ 7.41 (s, br, 1H), 7.13-7.02 (m, 3H), 6.99-6.93 (m, 2H), 6.63-6.57 (m, 2H), 6.48-6.42 (m, 2H), 3.74 (s, 3H), 3.67 (s, 3H).

### Example 15

### 1-Ethyl-4-[(4-methoxyphenyl)amino]-3-phenyl-5-thioxo-1,5-dihydro-2H-pyrrol-2-one

A mixture of 1-ethyl-3-[(4-methoxyphenyl)amino]-4-phenyl-1H-pyrrole-2,5-dione (0.40 mmol, 128 mg) and Lawesson's reagent (0.40 mmol, 161 mg) in toluene (2.0 mL) was heated in a microwave reactor at 160°C for 15 min.. The solvent was evaporated at reduced pressure. The residue was redissolved in THF and purified by HPLC (95% 0.1M ammonium acetate buffer: 5% CH₃CN → 100% CH₃CN) to give 95 mg (71 %) of the title compound. ¹H NMR (400 MHz, CDCl₃) δ 7.80 (s, br, 1H), 7.13-7.04 (m, 3H), 7.01-6.97 (m, 2H), 6.65-6.60 (m 2H), 6.56-6.51 (m, 2H), 4.01 (q, J=7.1 Hz, 2H), 3.69 (s, 3H), 1.31 (t, J=7.1 Hz, 3H).

### Example 16

### 1-Ethyl-3-[(4-methoxyphenyl)amino]4-phenyl-1H-pyrrole-2,5-dithione

A mixture of 1-ethyl-3-[(4-methoxyphenyl)amino]-4-phenyl-1H-pyrrole-2,5-dione (0.09 mmol, 29 mg) and Lawesson's reagent (0.18 mmol, 73 mg) in toluene (2.5 mL) was heated in a microwave reactor at 180°C for 60 min.. The solvent was evaporated at reduced pressure. The residue was redissolved in THF and purified by HPLC (95% 0.1M ammonium acetate buffer: 5% CH₃CN → 100% CH₃CN) to give 15 mg (48%) of the title compound. ¹H NMR (400 MHz, CDCl₃) δ 7.42 (s, br, 1H), 7.12-7.02 (m, 3H), 6.98-6.93 (m, 2H), 6.63-6.57 (m 2H), 6.47-6.42 (m, 2H), 4.41 (q, J=7.1 Hz, 2H), 3.67 (s, 3H), 1.31 (t, J=7.1 Hz, 3H).

### Example 17

### 1-[(6-Aminopyridin-3-yl)methyl]-4-{[4-(difluoromethoxy)phenyl]amino}-3-phenyl-5-thioxo-1,5-dihydro-2H-pyrrol-2-one

A mixture of 1-[(6-aminopyridin-3-yl)methyl]-3-{[4-(difluoromethoxy)phenyl]amino}-4-phenyl-1*H*-pyrrole-2,5-dione (128 mg, 0.29 mmol) and Lawesson's reagent (119 mg, 0.29 mmol) in toluene (4 mL) was heated in a microwave reactor at 150°C for 35 min.. The mixture was evaporated to dryness. Chromatography of the residue on a column (Isolute® FLASH SI, 70 g/150 mL), using CH₂Cl₂ and then CH₃OH/ CH₂Cl₂ (2:98, then 4:96) as eluant, gave a mixture. Re-chromatography of the mixture on a column (Isolute® SI, 5g/25 mL), using CH₂Cl₂ and then CH₃CN/ CH₂Cl₂ (10:90 and then 20:80) as eluant, gave 51 mg (38%) of the title compound. ¹H NMR (400 MHz, CDCl₃) δ 8.23 (d, J=2 Hz, 1H), 7.73 (bs, 1H), 7.59 (dd, J =8.5, 2 Hz, 1H), 7.15-7.06 (m, 3H), 6.99-6.96 (m, 2H), 6.77-6.73 (m, 2H), 6.66-6.62 (m, 2H), 6.44 (d, J=8.5 Hz, 1H), 6.33 (t, J=74 Hz, 1H), 4.98 (s, 2H) and 4.46 (bs, 2H).

### Example 18

### 1-[(6-Aminopyridin-3-yl)methyl]-3-{[4-(difluoromethoxy)phenyl]amino}-4-phenyl-1H-pyrrole-2,5-dithione

A mixture of 1-[(6-aminopyridin-3-yl)methyl]-3-{[4-(difluoromethoxy)phenyl]amino}-4-phenyl-1*H*-pyrrole-2,5-dione (128 mg, 0.29 mmol) and Lawesson's reagent (119 mg, 0.29 mmol) in toluene (4 mL) was heated in a microwave reactor at 150°C for 35 min.. The mixture was evaporated to dryness. Chromatography of the residue on a column (Isolute® FLASH SI, 70 g/150 mL), using CH₂Cl₂ and then CH₃OH / CH₂Cl₂ (2:98, then 4:96) as eluant, gave an oil mixture. Re-chromatography of the oil on a column (Isolute® SI, 5g/25 mL), using CH₂Cl₂ and then CH₃CN/ CH₂Cl₂ (10:90 and then 20:80) as eluant, gave two products. One of them was further purified by column chromatography (Isolute® SI 1g/6 mL, eluted with CH₃CN/ CH₂Cl₂ (10:90)) to give 5 mg (4%) of the title compound. ¹H NMR (400 MHz, CDCl₃) δ 8.25 (d, J=2 Hz, 1H), 7.60 (dd, J =8.5, 2 Hz, 1H), 7.33 (bs, 1H), 7.13-7.03 (m, 3H), 6.95-6.92(m, 2H), 6.68-6.62 (m, 4H), 6.43 (d, J= 8.4 Hz, 1H), 6.31(t, J=74 Hz, 1H), 5.44 (s, 2H) and 4.41 (bs, 2H).

### Example 19

### 1-[(6-Aminopyridin-3-yl)methyl]-4-[(4-morpholin-4-ylphenyl)amino]-3-phenyl-5-thioxo-1,5-dihydro-2H-pyrrol-2-one

A mixture of 1-[(6-aminopyridin-3-yl)methyl]-3-[(4-morpholin-4-ylphenyl)amino]-4-phenyl-1*H*-pyrrole-2,5-dione (130 mg, 0.28 mmol) and Lawesson's reagent (115 mg, 0.28 mmol) in toluene (50 mL) under nitrogen atmosphere was refluxed for 3 days and then evaporated to dryness. Chromatography of the residue on a column (Isolute® FLASH SI, 50g/150 mL), using CH₂Cl₂ and then CH₃OH / CH₂Cl₂ (2:98, then 4:96 and then 8:92) as eluant, gave a mixture. Re-chromatography of the mixture on a column (Isolute® SI, 20g/70 mL), using CH₃CN/ CH₂Cl₂ (20:80, then 50:50) as eluant, gave 47 mg (35%) of the title compound. ¹H NMR (400 MHz, CDCl₃) δ 8.23 (d, J= 2 Hz, 1H), 7.78 (s, 1H), 7.58 (dd, J= 8, 2 Hz, 1H), 7.09-7.03 (m, 3H), 6.97-6.94 (m, 2H), 6.58-6.49 (m, 4H), 6.42 (d, J= 8 Hz, 1H), 4.97 (s, 2H), 4.47 (bs, 2H) 3.81-3.78(m, 4H) and 2.99-2.97 (m, 4H).

### Example 20

### 1-[(6-aminopyridin-3-yl)methyl]-3-[(4-morpholin-4-ylphenyl)amino]-4-phenyl-1H-pyrrole-2,5-dithione

A mixture of 1-[(6-aminopyridin-3-yl)methyl]-3-[(4-morpholin-4-ylphenyl)amino]-4-phenyl-1*H*-pyrrole-2,5-dione (140 mg, 0.31 mmol) and Lawesson's reagent (249 mg, 0.61 mmol) in toluene (4.5 mL) was heated in a microwave reactor at 150°C for 20 min. and then evaporated to dryness. Chromatography of the residue on a column (Isolute® FLASH SI, 50 g/150 mL), using CH₂Cl₂ and then CH₃OH/ CH₂Cl₂ (2:98, then 4:96 and then 8:92) as eluant, gave a mixture containing 1-[(6-aminopyridin-3-yl)methyl]-4-[(4-morpholin-4-ylphenyl)amino]-3-phenyl-5-thioxo-1,5-dihydro-2*H-*pyrrol-2-one and trace amount of desired product. This mixture was treated with Lawesson's reagent (30 mg) in toluene (4 mL) and heated in a microwave reactor at 160°C for 30 min., and then evaporated to dryness. Chromatography of the residue on a column (Isolute® FLASH SI, 20g/70 mL), using CH₂Cl₂ and then CH₃OH/ CH₂Cl₂ (2:98, and then 4:96) as eluant, gave 4 mg product. Re-chromatography of it on a column (Isolute® FLASH SI, 20g/70 mL), using CH₂Cl₂ and CH₃CN/ CH₂Cl₂ (25:75, then 50:50) as eluant, gave 3 mg product. Re-chromatography of it once again on a column (Isolute® SI, 1g/6 mL), using CH₂Cl₂ and then CH₃OH/ CH₂Cl₂ (1:99) as eluant, gave 2 mg (1%) of the title compound. ¹H NMR (400 MHz, CDCl₃): δ 8.25 (d, J= 2 Hz, 1H), 7.63 (dd, J= 8.5, 2.2 Hz, 1H), 7.44 (bs, 1H), 7.10-7.01 (m, 3H), 6.95-6.92 (m, 2H), 6.57-6.53 (m, 2H), 6.44-6.41 (m, 3H), 5.44 (s, 2H), 4.44 (bs, 2H) 3.81-3.78(m, 4H) and 2.99-2.96 (m, 4H).

### BIOLOGICAL ACTIVITY

### CO-ACTIVATOR RECRUITMENT ASSAY

The Ligand Binding Domain (LBD) of human LXRalpha (amino acid 205-447) and LXRbeta (amino acid 216-461) was produced by recombinant techniques in E coli. A fragment of the human Steroid Receptor Co-Activator-1 (SRC-1) was produced as a synthetic peptide. An anti-6His-antibody coupled with Europium (Eu³⁺) was used to recognize the His-tag on the LXR-LBD and Allophycocyanin (APC) coupled to streptavidin was used to recognize the biotinylated SRC-1. Agonist binding to LXRalpha or LXRbeta enhances the affinity of LXR towards SRC-1 and thereby brings Eu³⁺ and APC in close proximity. Eu³⁺ is excited at 337 nm and emitts light at 620 nm. This emission, when in close proximity, excites APC to emit light at 665 nm.

Dilution plates with compounds in DMSO were further diluted in buffer {20mM [Tris(hydroxymethyl)aminomethane] pH 7.5, 0.125% CHAPS {3-[(3-Cholamidopropyl)dimethylammonio]-1-propanesulfonate}, 2mM DTT (Dithiothreitol) and 0.05% BSA (Bovine Serum Albumin)} in order to reduce DMSO concentration, 0.5 µl to 13.5 µl. To this, 6 µl assay mix was added and the plates (384-well V-groove plates) were incubated at room temperature for 60 to 80 min. The assay mix has the following final concentrations; LXRalpha mix: 0.06 µg/ mL Eu-labelled anti-6x His Ab, 1.15µg/ mL Streptavidin APC, 30 nM SRC-1 peptide and 0.9 µg/ mL LXRalpha in buffer and LXRbeta mix; 0.06 µg/ mL Eu-labelled anti-6x His Ab, 1.15µg/ mL Streptavidin APC, 90 nM SRC-1 peptide and 0.2 µg/ mL LXRbeta in buffer. Time-resolved fluorescence readings were done in a Wallac Victor reader at 665 nm followed by reading at 615 nm. The LXR ligand, 22-R Hydroxycholesterol at 50µM was used as the 100% control.

### TRANSACTIVATION ASSAY

Expression vectors were prepared by inserting the ligand binding domain cDNA (complementary DNA) of human LXRalpha (amino acid 205-447) and LXRbeta (amino acid 216-461) in frame with, 3' to the yeast GAL4 transcription factor DNA binding domain and the nuclear localization signal from the T-antigen of Polyoma Virus in the eucaryotic expression vector pSG5 (Stratagene). The resulting expression vectors pSGGAL-LXRalpha and pSGGAL-LXRbeta were used in cotransfection experiments together with the pGL3 luciferase reporter plasmid containing a minimal SV40 promoter and five copies of the UAS GAL4 recognition site. 2.5 µg pSGGAL-LXRalpha or beta were mixed with 25 µg pGL3 5xUAS and 22.5 µg pBluscript in 0.95 mL ice cold PBS containing approx. 4-9 milj. U2/OS osteosarcoma cells. After a five minute incubation on ice the cell/DNA mixture was electroporated in 0.4 cm cuvettes at 960 µF, 230 V using a BioRad electroporator and diluted to 0.32 milj cells / mL in complete DMEM (Dulbecco's Modified Eagle Medium) medium (Gibco 31966-021). Cells from at least two electroporations were pooled in order to avoid variations between different electroportations. 25 µl diluted, electroporated cells, were seeded onto 384-well plates (0.8 x 10⁴ cells/well) and the cells were allowed to adhere for 2 h at 37°C, 5% CO₂ in a cell culture incubator. Dilution plates with compounds in DMSO were further diluted in DMEM w/o phenol red (Gibco 11880-028) including 10% FBS (Foetal Bovine Serum), 1% PEST (Penicillin Streptomycin), 20mM Hepes, 2mM L-Glutamine and 0.36% Glucose (2.5 µl to 97.5 µl) in order to reduce DMSO concentration. 7 µl of this was added to the electroporated cells in 384-well plates and incubation was continued for 48 h in a cell culture incubator, after which cells were lysed by adding 32 µl/well LucLite luciferase substrate. Luciferase activity was measured using the "Luminescence 384 protocol" in the Wallac Victor reader after 15 min. incubation at room temperature. The LXR ligand, Tularik T0901317, at 1µM was used as the 100% control.

The compounds of formula I have an EC₅₀ of less than 50µmol/l for LXRalpha and/or beta in coactivator recruitment assays and/or reporter gene assays. For example, the compounds of Examples 7 and 18 were EC₅₀'s of 0.09 µmol/l and 0.14 µmol/l in coactivator recruitment assays, respectively.

In addition the compounds of the present invention exhibit improved physical and/or chemical and/or DMPK (Drug Metabolism and Pharmacokinetic) properties, for example they exhibit improved metabolic stability *in vitro*, and/or exhibit favourable pharmacological effects *in vivo*. The compounds also have a promising toxicological profile.

## Claims

1. A compound of Formula I 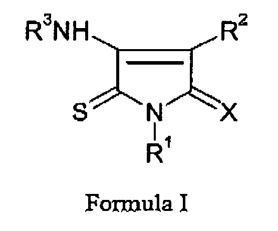 wherein:
**R¹** is selected from phenyl(1-4C)alkyl wherein the phenyl is optionally substituted by (1-4C)alkoxycarbonyl or a group of formula NR^{a}R^{b} in which R^{a} and R^{b} independently represent H or (1-4C)alkyl; heteroaryl(1-4C)alkyl, where the term heteroaryl means pyridyl, furyl or isoxazolyl, wherein the heteroaryl is optionally substituted by (1-4C)alkyl or a group of formula NR^{a}R^{b} in which R^{a} and R^{b} independently represent H or (1-4C)alkyl; or a (1-6C)alkyl group which is optionally substituted by one or more of the following: fluoro, (1-4C)alkoxycarbonyl, (1-3C)alkylthio or (1-3C)alkoxy optionally substituted by one or more fluoro;
**R²** is phenyl;
**R³** is selected from phenyl, indolyl or benzofuranyl each optionally substituted by one or more of the following: (1-3C)alkanoyl, (1-4C)alkoxy optionally substituted by one or more fluoro; (1-3C)alkylthio; or a group of formula NR^{a}R^{b} in which R^{a} and R^{b} independently represent H, (1-3C)alkyl or (1-3C)alkanoyl or R^{a} and R^{b} together with the nitrogen atom to which they are attached represent morpholino;
**X** is O or S;
or a pharmaceutically acceptable salt or solvate thereof, or a solvate of such a salt.

2. A compound according to claim 1 wherein **X** is O.

3. A compound according to claim 1 wherein **X** is S.

4. A compound according to any previous claim in which **R¹** is selected from methyl, ethyl, propyl, butyl, 2-methoxyethyl, 2,2,2-trifluoroethyl, benzyl, 4-pyridylmethyl, 3-pyridylmethyl or 6-amino-3-pyridylmethyl.

5. A compound according to any previous claim in which **R³** is 4-methoxyphenyl, 4-difluoromethoxyphenyl or 4-morpholinophenyl.

6. A compound according to any of claims 1-3 wherein **R¹** is selected from methyl, ethyl, 2,2,2-trifluoroethyl, benzyl, 3-pyridylmethyl or 6-amino-3-pyridylmethyl;
**R²** is phenyl;
**R³** is selected from 4-methoxyphenyl, 4-difluoromethoxyphenyl or 4-morpholinophenyl;
**X** is O or S.

7. A compound according to any of claims 1-3 wherein **R¹** is selected from ethyl, 2,2,2-trifluoroethyl, benzyl, 3-pyridylmethyl or 6-amino-3-pyridylmethyl;
**R²** is phenyl;
**R³** is selected from 4-methoxyphenyl, 4-difluoromethoxyphenyl or 4-morpholinophenyl;
**X** is O or S.

8. A compound according to any of claims 1-3 wherein **R¹** is selected from methyl, ethyl, 2,2,2-trifluoroethyl, 2-pyridylmethyl, 3-pyridylmethyl or 4-pyridylmethyl;
**R²** is phenyl;
**R³** is selected from 4-methoxyphenyl;
**X** is O or S.

9. A compound according to any of claims 1-3 wherein **R¹** is selected from 2-methoxyethyl or 6-amino-3-pyridylmethyl;
**R²** is phenyl;
**R³** is selected from 4-methoxyphenyl or 4-difluoromethoxyphenyl;
**X** is O or S.

10. A compound of Formula I according to claim 1 selected from one or more of the following:
1-(2-Methoxyethyl)-4-[(4-methoxyphenyl)amino]-3-phenyl-5-thioxo-1,5-dihydro-2H-pyrrol-2-one;
1-(2-Methoxyethyl)-3-[(4-methoxyphenyl)amino]-4-phenyl-1H-pyrrole-2,5-dithione;
4-[(4-Methoxyphenyl)amino]-3-phenyl-1-(pyridin-3-ylmethyl)-5-thioxo-1,5-dihydro-2H-pyrrol-2-one;
3-[(4-Methoxyphenyl)amino]-4-phenyl-1-(pyridin-3-ylmethyl)-1H-pyrrole-2,5-dithione;
4-[(4-Methoxyphenyl)aminol-3-phenyl-1-(pyridin-4-ylmethyl)-5-thioxo-1,5-dihydro-2H-pyrrol-2-one;
3-[(4-Methoxyphenyl)amino]-4-phenyl-1-(pyridin-4-ylmethyl)-1H-pyrrole-2,5-dithione;
1-Butyl-4-[(4-methoxyphenyl)amino]-3-phenyl-5-thioxo-1,5-dihydro-2H-pyrrol-2-one;
1-Butyl-3-[(4-methoxyphenyl)amino]-4-phenyl-1H-pyrrole- 2,5-dithione;
4-[(4-Methoxyphenyl)amino]-3-phenyl-5-thioxo-1-(2,2,2-trifluoroethyl)-1,5-dihydro-2H-pyrrol-2-one;
3-[(4-Methoxyphenyl)amino]-4-phenyl-1-(2,2,2-trifluoroethyl)-1H-pyrrole-2,5-dithione;
1-Benzyl-4-[(4-methoxyphenyl)amino]-3-phenyl-5-thioxo-1,5-dihydro-2H-pyrrol-2-one;
1-Benzyl-3-[(4-methoxyphenyl)amino]-4-phenyl-1H-pyrrole-2,5-dithione;
4-[(4-Methoxyphenyl)amino]-1-methyl-3-phenyl-5-thioxo-1,5-dihydro-2H-pyrrrol-2-one;
3-[(4-Methoxyphenyl)amino]-1-methyl-4-phenyl-1H-pyrrole-2,5-dithione;
1-Ethyl-4-[(4-methoxyphenyl)amino]-3-phenyl-5-thioxo-1,5-dihydro-2H-pyrrol-2-one;
1-Ethyl-3-[(4-methoxyphenyl)amino]-4-phenyl-1H-pyrrole-2,5-dithione;
1-[(6-Aminoppridin-3-yl)methyl]-4-{[4-(difluoromethoxy)phenyl]amino}-3-phenyl-5-thioxo-1,5-dihydro-2H-pyrrol-2-one;
1-[(6-Aminopyridin-3-yl)methyl]-3-{[4-(difluoromethoxy)phenyl]amino}-4-phenyl-1H-pyrrole-2,5-dithione;
1-[(6-Aminopyridin-3-yl)methyl]-4-[(4-morpholin-4-ylphenyl)amino]-3-phenyl-5-thioxo-1,5-dihydro-2H-pyrrol-2-one;
1-[(6-Aminopyridin-3-yl)methyl]-3-[(4-morpholin-4-ylphenyl)amino]-4-phenyl-1H-pyrrole-2,5-dithione;
or a pharmaceutically acceptable salt or solvate thereof, or a solvate of such a salt.

11. A process for the preparation of a compound according to any one of claims 1-10, wherein **R¹, R²** and **R³** are as defined in claim 1, comprising the step of reacting a compound of formula II, 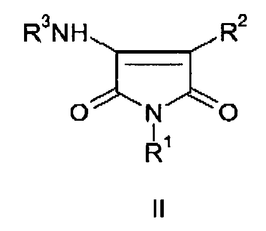 wherein R² and R³ are as defined in claim 1, with a sulphurating agent in the presence of an inert organic liquid at a temperature in the range of 0°C to 200°C.

12. A pharmaceutical formulation comprising a compound according to any one of claims 1-10, or a pharmaceutically acceptable salt thereof, in admixture with pharmaceutically acceptable adjuvants, diluents and/or carriers.

13. A compound according to any one of claims 1-10, or a pharmaceutically acceptable salt thereof, for use as a medicament in therapy.

14. The use of a compound according to any one of claims 1-10 in the manufacture of a medicament for the treatment and/or prophylaxis of cardiovascular disease.

15. The use of a compound according to any one of claims 1-10 in the manufacture of a medicament for the treatment and/or prophylaxis of atherosclerosis.

16. The use of a compound according to any one of claims 1-10 in the manufacture of a medicament for the treatment and/or prophylaxis of hypercholesterolemia.

17. The use of a compound according to any one of claims 1-10 in the manufacture of a medicament for the treatment and/or prophylaxis of conditions associated with a need for improving reverse cholesterol transport.

18. The use of a compound according to anyone of claims 1-10 in the manufacture of a medicament for the treatment and/or prophylaxis of conditions associated with a need for decreasing intestinal cholesterol absorption.

19. The use of a compound according to any one of claims 1-10 in the manufacture of a medicament for the treatment and/or prophylaxis of conditions associated with a need for increasing HDL-cholesterol levels.

20. The use of a compound according to any one of claims 1-10 in the manufacture of a medicament for the treatment and/or prophylaxis of conditions associated with a need for decreasing LDL-cholesterol levels.

21. The usc of a compound according to any one of claims 1-10 in the manufacture of a medicament for the treatment and/or prophylaxis of inflammatory conditions.

22. The use of a compound according to any one of claims 1-10 in the manufacture of a medicament for the treatment and/or prophylaxis of Alzheimer's disease.

23. The use of a compound according to any one of claims 1-10 in the manufacture of a medicament for the treatment and/or prophylaxis of arteriosclerosis.

24. The use of a compound according to any one of claims 1-10 in the manufacture of a medicament for the treatment and/or prophylaxis of type 2 diabetes.

25. The use of a compound according to any one of claims 1-10 in the manufacture of a medicament for the treatment and/or prophylaxis of lipid disorders (dyslipidemia) whether or not associated with insulin resistance.

26. A pharmaceutical formulation for use in the treatment or prophylaxis of conditions associated with a need for modulation of the nuclear hormone receptors LXR α and/or β, comprising a compound according to any one of claims 1-10 as active ingredient in admixture with a pharmaceutically acceptable adjuvant, diluent or carrier.

27. A pharmaceutical composition comprising a compound as claimed in any one of claims 1-10 combined with another therapeutic agent that is useful in the treatment of conditions or disorders associated with the development and progress of atherosclerosis such as hypertension, dyslipidemias, hyperlipidaemias, hypercholesterolemias, type 2 diabetes, inflammation, obesity as well as conditions associated with a need for improving reverse cholesterol transport and/or decreasing intestinal cholesterol absorption.

## Patentansprüche

1. Verbindung der Formel I 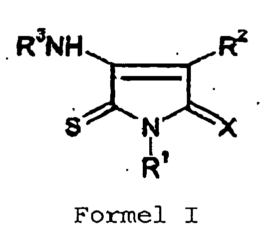 worin:
R¹ unter Phenyl-C₁₋₄-alkyl, worin das Phenyl gegebenenfalls durch C₁₋₄-Alkoxycarbonyl oder eine Gruppe der Formel NR^{a}R^{b}, in der R^{a} und R^{b} unabhängig voneinander für H oder C₁₋₄-Alkyl stehen, substituiert ist; Heteroaryl-C₁₋₄-alkyl, wobei der Begriff Heteroaryl Pyridyl, Furyl oder Isoxazolyl bedeutet, worin das Heteroaryl gegebenenfalls durch C₁₋₄-Alkyl oder eine Gruppe der Formel NR^{a}R^{b}, in der R^{a} und R^{b} unabhängig voneinander für H oder C₁₋₄-Alkyl stehen, substituiert ist; oder einer C₁₋₆-Alkylgruppe, die gegebenenfalls ein- oder mehrfach durch Fluor, C₁₋₄-Alkoxycarbonyl, C₁₋₃-Alkylthio oder C₁₋₃-Alkoxy, gegebenenfalls ein- oder mehrfach substituiert durch Fluor, substituiert ist; ausgewählt ist;
R² für Phenyl steht;
R³ unter Phenyl, Indolyl oder Benzofuranyl, jeweils gegebenenfalls ein- oder mehrfach substituiert durch C₁₋₃-Alkanoyl, C₁₋₄-Alkoxy, gegebenenfalls ein- oder mehrfach substituiert durch Fluor; C₁₋₃-Alkylthio oder eine Gruppe der Formel NR^{a}R^{b}, in der R^{a} und R^{b} unabhängig voneinander für H, C₁₋₃-Alkyl oder C₁₋₃-Alkanoyl stehen oder R^{a} und R^{b} gemeinsam mit dem Stickstoffatom, an das sie gebunden sind, für Morpholino stehen, ausgewählt ist;
X für O oder S steht;
oder ein pharmazeutisch annehmbares Salz oder Solvat davon oder ein Solvat eines derartigen Salzes.

2. Verbindung nach Anspruch 1, worin X für O steht.

3. Verbindung nach Anspruch 1, worin X für S steht.

4. Verbindung nach einem der vorhergehenden Ansprüche, worin R¹ unter Methyl, Ethyl, Propyl, Butyl, 2-Methoxyethyl, 2,2,2-Trifluorethyl, Benzyl, 4-Pyridylmethyl, 3-Pyridylmethyl oder 6-Amino-3-pyridylmethyl ausgewählt ist.

5. Verbindung nach einem der vorhergehenden Ansprüche, worin R³ für 4-Methoxyphenyl, 4-Difluormethoxyphenyl oder 4-Morpholinophenyl steht.

6. Verbindung nach den Ansprüchen 1-3, worin R¹ unter Methyl, Ethyl, 2,2,2-Trifluorethyl, Benzyl, 3-Pyridylmethyl oder 6-Amino-3-pyridylmethyl ausgewählt ist;
R² für Phenyl steht;
R³ unter 4-Methoxyphenyl, 4-Difluormethoxyphenyl oder 4-Morpholinophenyl ausgewählt ist;
X für O oder S steht.

7. Verbindung nach den Ansprüchen 1-3, worin R¹ unter Ethyl, 2,2,2-Trifluorethyl, Benzyl, 3-Pyridylmethyl oder 6-Amino-3-pyridylmethyl ausgewählt ist;
R² für Phenyl steht;
R³ unter 4-Methoxyphenyl, 4-Difluormethoxyphenyl oder 4-Morpholinophenyl ausgewählt ist;
X für O oder S steht.

8. Verbindung nach den Ansprüchen 1-3, worin R¹ unter Methyl, Ethyl, 2,2,2-Trifluorethyl, 2-Pyridylmethyl, 3-Pyridylmethyl oder 4-Pyridylmethyl ausgewählt ist;
R² für Phenyl steht;
R³ unter 4-Methoxyphenyl ausgewählt ist;
X für O oder S steht.

9. Verbindung nach den Ansprüchen 1-3, worin R¹ unter 2-Methoxyethyl oder 6-Amino-3-pyridylmethyl ausgewählt ist;
R² für Phenyl steht;
R³ unter 4-Methoxyphenyl oder 4-Difluormethoxyphenyl ausgewählt ist;
X für O oder S steht.

10. Verbindung der Formel I nach Anspruch 1, ausgewählt unter einer oder mehreren der folgenden Verbindungen:
1-(2-Methoxyethyl)-4-[(4-methoxyphenyl)amino]-3-phenyl-5-thioxo-1,5-dihydro-2H-pyrrol-2-on;
1-(2-Methoxyethyl)-3-[(4-methoxyphenyl)amino]-4-phenyl-1H-pyrrol-2,5-dithion;
4-[(4-Methoxyphenyl)amino]-3-phenyl-1-(pyridin-3-ylmethyl)-5-thioxo-1,5-dihydro-2H-pyrrol-2-on;
3-[(4-Methoxyphenyl)amino]-4-phenyl-1-(pyridin-3-ylmethyl)-1H-pyrrol-2,5-dithion;
4-[(4-Methoxyphenyl)amino]-3-phenyl-1-(pyridin-4-ylmethyl)-5-thioxo-1,5-dihydro-2H-pyrrol-2-on;
3-[(4-Methoxyphenyl)amino]-4-phenyl-1-(pyridin-4-ylmethyl)-1H-pyrrol-2,5-dithion;
1-Butyl-4-[(4-methoxyphenyl)amino]-3-phenyl-5-thioxo-1,5-dihydro-2H-pyrrol-2-on;
1-Butyl-3-[(4-methoxyphenyl)amino]-4-phenyl-1H-pyrrol-2,5-dithion;
4-[(4-Methoxyphenyl)amino]-3-phenyl-5-thioxo-1-(2,2,2-trifluorethyl)-1,5-dihydro-2H-pyrrol-2-on;
3-[(4-Methoxyphenyl)amino]-4-phenyl-1-(2,2,2-trifluorethyl)-1H-pyrrol-2,5-dithion;
1-Benzyl-4-[(4-methoxyphenyl)amino]-3-phenyl-5-thioxo-1,5-dihydro-2H-pyrrol-2-on;
1-Benzyl-3-[(4-methoxyphenyl)amino]-4-phenyl-1H-pyrrol-2,5-dithion;
4-[(4-Methoxyphenyl)amino]-1-methyl-3-phenyl-5-thioxo-1,5-dihydro-2H-pyrrol-2-on;
3-[(4-Methoxyphenyl)amino]-1-methyl-4-phenyl-1H-pyrrol-2,5-dithion;
1-Ethyl-4-[(4-methoxyphenyl)amino]-3-phenyl-5-thioxo-1,5-dihydro-2H-pyrrol-2-on;
1-Ethyl-3-[(4-methoxyphenyl)amino]-4-phenyl-1H-pyrrol-2,5-dithion;
1-[(6-Aminopyridin-3-yl)methyl]-4-{[4-(difluormethoxy)phenyl]amino}-3-phenyl-5-thioxo-1,5-dihydro-2H-pyrrol-2-on;
1-[(6-Aminopyridin-3-yl)methyl]-3-{[4-(difluormethoxy)phenyl]amino}-4-phenyl-1H-pyrrol-2,5-dithion;
1-[(6-Aminopyridin-3-yl)methyl]-4-[(4-morpholin-4-ylphenyl)amino]-3-phenyl-5-thioxo-1,5-dihydro-2H-pyrrol-2-on;
1-[(6-Aminopyridin-3-yl)methyl]-3-[(4-morpholin-4-ylphenyl)amino]-4-phenyl-1H-pyrrol-2,5-dithion;
oder ein pharmazeutisch annehmbares Salz oder Solvat davon oder ein Solvat eines derartigen Salzes.

11. Verfahren zur Herstellung einer Verbindung nach einem der Ansprüche 1-10, worin R¹, R² und R³ die in Anspruch 1 angegebene Bedeutung besitzen, bei dem man eine Verbindung der Formel II 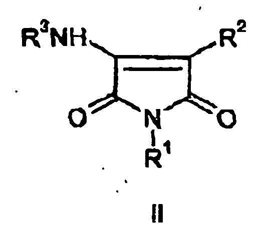 worin R² und R³ die in Anspruch 1 angegebene Bedeutung besitzen, in Gegenwart einer inerten organischen Flüssigkeit bei einer Temperatur im Bereich von 0°C bis 200°C mit einem Sulfurierungsmittel umsetzt.

12. Pharmazeutische Formulierung, enthaltend eine Verbindung nach einem der Ansprüche 1-10 oder ein pharmazeutisch annehmbares Salz davon in Abmischung mit pharmazeutisch annehmbaren Hilfsstoffen, Verdünnungsmitteln und/oder Trägern.

13. Verbindung nach einem der Ansprüche 1-10 oder ein pharmazeutisch annehmbares salz davon zur Verwendung als Arzneimittel bei der Therapie.

14. Verwendung einer Verbindung nach einem der Ansprüche 1-10 bei der Herstellung eines Arzneimittels zur Behandlung und/oder Prophylaxe von Herz-Kreislauf-Erkrankung.

15. Verwendung einer Verbindung nach einem der Ansprüche 1-10 bei der Herstellung eines Arzneimittels zur Behandlung und/oder Prophylaxe von Atherosklerose.

16. Verwendung einer Verbindung nach einem der Ansprüche 1-10 bei der Herstellung eines Arzneimittels zur Behandlung und/oder Prophylaxe von Hypercholesterinämie.

17. Verwendung einer Verbindung nach einem der Ansprüche 1-10 bei der Herstellung eines Arzneimittels zur Behandlung und/oder Prophylaxe von Leiden, die mit einem Bedarf an der Verbesserung des reversen Cholesterintransports assoziiert sind.

18. Verwendung einer Verbindung nach einem der Ansprüche 1-10 bei der Herstellung eines Arzneimittels zur Behandlung und/oder Prophylaxe von Leiden, die mit einem Bedarf an der Verringerung der intestinalen Cholesterinresorption assoziiert sind.

19. Verwendung einer Verbindung nach einem der Ansprüche 1-10 bei der Herstellung eines Arzneimittels zur Behandlung und/oder Prophylaxe von Leiden, die mit einem Bedarf an der Erhöhung der HDL-Cholesterin-Spiegel assoziiert sind.

20. Verwendung einer Verbindung nach einem der Ansprüche 1-10 bei der Herstellung eines Arzneimittels zur Behandlung und/oder Prophylaxe von Leiden, die mit einem Bedarf an der Verringerung der LDL-Cholesterin-Spiegel assoziiert sind.

21. Verwendung einer Verbindung nach einem der Ansprüche 1-10 bei der Herstellung eines Arzneimittels zur Behandlung und/oder Prophylaxe von entzündlichen Leiden.

22. Verwendung einer Verbindung nach einem der Ansprüche 1-10 bei der Herstellung eines Arzneimittels zur Behandlung und/oder Prophylaxe von Alzheimer-Krankheit.

23. Verwendung einer Verbindung nach einem der Ansprüche 1-10 bei der Herstellung eines Arzneimittels zur Behandlung und/oder Prophylaxe von Arteriosklerose.

24. Verwendung einer Verbindung nach einem der Ansprüche 1-10 bei der Herstellung eines Arzneimittels zur Behandlung und/oder Prophylaxe von Typ-2-Diabetes.

25. Verwendung einer Verbindung nach einem der Ansprüche 1-10 bei der Herstellung eines Arzneimittels zur Behandlung und/oder Prophylaxe von Lipidstörungen (Dyslipidämie), ob mit Insulinresistenz assoziiert oder nicht.

26. Pharmazeutische Formulierung zur Verwendung bei der Behandlung oder Prophylaxe von Leiden, die mit einem Bedarf an der Modulierung der nukleären Hormonrezeptoren LXR α und/oder β assoziiert sind, enthaltend eine Verbindung nach einem der Ansprüche 1-10 als Wirkstoff in Abmischung mit einem pharmazeutisch annehmbaren Hilfsstoff, Verdünnungsmittel oder Träger.

27. Pharmazeutische Zusammensetzung, enthaltend eine Verbindung nach einem der Ansprüche 1-10 in Kombination mit einem anderen Therapeutikum, das zur Verwendung bei der Behandlung von Leiden oder Störungen, die mit der Entwicklung und dem Fortschreiten von Atherosklerose assoziiert sind, wie Hypertonie, Dyslipidämien, Hyperlipidämien, Hypercholesterinämien, Typ-2-Diabetes, Entzündung, Adipositas, sowie Leiden, die mit einem Bedarf an der Verbesserung des reversen Cholesterintransports und/oder der Verringerung der intestinalen Cholesterinresorption assoziiert sind, geeignet ist.

## Revendications

1. Composé de formule I 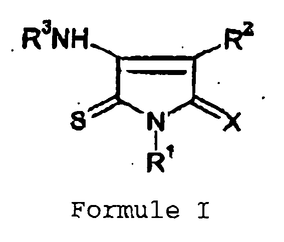 dans laquelle :
R¹ est choisi parmi phényl(1-4C)alkyle où le phényle est éventuellement substitué par (1-4C)alcoxycarbonyle ou un groupement de formule NR^{a}R^{b} dans laquelle R^{a} et R^{b} représentent indépendamment H ou (1-4C)alkyle ; hétéroaryl(1-4C)alkyle, où le terme hétéroaryle signifie pyridyle, furyle ou isoxazolyle, où l'hétéroaryle est éventuellement substitué par (1-4C)alkyle ou un groupement de formule NR^{a}R^{b} dans laquelle R^{a} et R^{b} représentent indépendamment H ou (1-4C)alkyle ; ou un groupement (1-6C)alkyle qui est éventuellement substitué par un ou plusieurs de ce qui suit : fluoro, (1-4C)alcoxycarbonyle, (1-3C)alkylthio ou (1-3C)alcoxy éventuellement substitué par un ou plusieurs fluoro ;
R² est phényle ;
R³ est choisi parmi phényle, indolyle ou benzofuranyle chacun éventuellement substitué par un ou plusieurs de ce qui suit : (1-3C)alcanoyle, (1-4C)alcoxy éventuellement substitué par un ou plusieurs fluoro ; (1-3C)alkylthio ; ou un groupement de formule NR^{a}R^{b} dans laquelle R^{a} et R^{b} représentent indépendamment H, (1-3C)alkyle ou (1-3C)alcanoyle ou R^{a} et R^{b} ensemble avec l'atome d'azote auquel ils sont liés représentent morpholino ;
X est O ou S ;
ou un sel ou un solvate pharmaceutiquement acceptable de celui-ci, ou un solvate d'un tel sel.

2. Composé selon la revendication 1, **caractérisé en ce que** X est O.

3. Composé selon la revendication 1, **caractérisé en ce que** X est S.

4. Composé selon l'une quelconque des revendications précédentes, **caractérisé en ce que** R¹ est choisi parmi méthyle, éthyle, propyle, butyle, 2-méthoxyéthyle, 2,2,2-trifluoroéthyle, benzyle, 4-pyridylméthyle, 3-pyridylméthyle ou 6-amino-3-pyridylméthyle.

5. Composé selon l'une quelconque des revendications précédentes, **caractérisé en ce que** R³ est 4-méthoxyphényle, 4-difluorométhoxyphényle ou 4-morpholinophényle.

6. Composé selon l'une quelconque des revendications 1 à 3, **caractérisé en ce que** R¹ est choisi parmi méthyle, éthyle, 2,2,2-trifluoroéthyle, benzyle, 3-pyridylméthyle ou 6-amino-3-pyridylméthyle ;
R² est phényle ;
R³ est choisi parmi 4-méthoxyphényle, 4-difluorométhoxyphényle ou 4-morpholinophényle ;
X est O ou S.

7. Composé selon l'une quelconque des revendications 1 à 3, **caractérisé en ce que** R¹ est choisi parmi éthyle, 2,2,2-trifluoroéthyle, benzyle, 3-pyridylméthyle ou 6-amino-3-pyridylméthyle ;
R^{a} est phényle ;
R³ est choisi parmi 4-méthoxyphényle, 4-difluorométhoxyphényle ou 4-morpholinophényle ;
X est O ou S.

8. Composé selon l'une quelconque des revendications 1 à 3, **caractérisé en ce que** R¹ est choisi parmi méthyle, éthyle, 2,2,2-trifluoroéthyle, 2-pyridylméthyle, 3-pyridylméthyle ou 4-pyridylméthyle ;
R² est phényle ;
R³ est choisi parmi 4-méthoxyphényle ;
X est O ou S.

9. Composé selon l'une quelconque des revendications 1 à 3, **caractérisé en ce que** R¹ est choisi parmi 2-méthoxyéthyle ou 6-amino-3-pyridylméthyle ;
R² est phényle ;
R³ est choisi parmi 4-méthoxyphényle ou 4-difluorométhoxyphényle ;
X est O ou S.

10. Composé de formule 1 selon la revendication 1, choisi parmi une ou plusieurs de ce qui suit :
la 1-(2-méthoxyéthyl)-4-[(4-méthoxyphényl)amino]-3-phényl-5-thioxo-1,5-dihydro-2H-pyrrol-2-one ;
la 1-(2-méthoxyéthyl)-3-[(4-méthoxyphényl)amino]-4-phényl-1H-pyrrol-2,5-dithione ;
la 4-[(4-méthoxyphényl)amino]-3-phényl-1-(pyridin-3-ylméthyl)-5-thioxo-1,5-dihydro-2H-pyrrol-2-one ;
la 3-[(4-méthoxyphényl)amino]-4-phényl-1-(pyridin-3-ylméthyl)-1H-pyrrol-2,5-dithione ;
la 4-[(4-méthoxyphényl)amino]-3-phényl-1-(pyridin-4-ylméthyl)-5-thioxo-1,5-dihydro-2H-pyrrol-2-one ;
la 3-[(4-méthoxyphényl)amino]4-phényl-1-(pyridin-4-ylméthyl)-1H-pyrrol-2,5-dithione ;
la 1-butyl-4-[(4-méthoxyphényl)amino]-3-phényl-5-thioxo-1,5-dihydro-2H-pyrrol-2-one ;
la 1-butyl-3-[(4-méthoxyphényl)amino]-4-phényl-1H-pyrrol-2,5-dithione ;
la 4-[(4-méthoxyphényl)amino]-3-phényl-5-thioxo-1-(2,2,2-trifluoroéthyl)-1,5-dihydro-2H-pyrrol-2-one ;
la 3-[(4-méthoxyphényl)amino]-4-phényl-1-(2,2,2-trifluoroéthyl)-1H-pyrrol-2,5-dithione ;
la 1-benzyl-4-[(4-méthoxyphényl)amino]-3-phényl-5-thioxo-1,5-dihydro-2H-pyrrol-2-one ;
la 1-benzyl-3-[(4-méthoxyphényl)amino]-4-phényl-1H-pyrrol-2,5-dithione ;
la 4-[(4-méthoxyphényl)amino]-1-méthyl-3-phényl-5-thioxo-1,5-dihydro-2H-pyrrol-2-one ;
la 3-[(4-méthoxyphényl)amino]-1-méthyl-4-phényl-1H-pyrrol-2,5-dithione ;
la 1-éthyl-4-[(4-méthoxyphényl)amino]-3-phényl-5-thioxo-1,5-dihydro-2H-pyrrol-2-one ;
la 1-éthyl-3-[(4-méthoxyphényl)amino]-4-phényl-1H-pyrrol-2,5-dithione ;
la 1-[(6-aminopyridin-3-yl)méthyl]-4-{[4-(difluorométhoxy)phényl]amino}-3-phényl-5-thioxo-1,5-dihydro-2H-pyrrol-2-one ;
la 1-[(6-aminopyridin-3-yl)méthyl]-3-{[4-(difluorométhoxy)phényl]amino}-4-phényl-1H-pyrrol-2,5-dithione ;
la 1-[(6-aminopyridin-3-yl)méthyl]-4-[(4-morpholin-4-ylphényl)amino]-3-phényl-5-thioxo-1,5-dihydro-2H-pyrrol-2-one ;
la 1-[(6-aminopyridin-3-yl)méthyl]-3-[(4-morpholin-4-ylphényl)amino]-4-phényl-1H-pyrrol-2,5-dithione ;
ou un sel ou un solvate pharmaceutiquement acceptable de celles-ci, ou un solvate d'un tel sel.

11. Procédé de préparation d'un composé selon l'une quelconque des revendications 1 à 10, **caractérisé en ce que** R¹, R² et R³ sont tels que définis dans la revendication 1, comprenant les étapes de mise en réaction d'un composé de formule II, 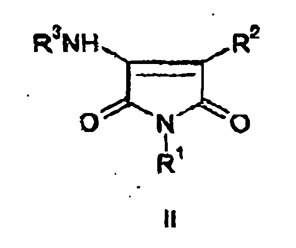 dans laquelle R² et R³ sont tels que définis dans la revendication 1, avec un agent de sulfuration en présence d'un liquide organique inerte, à une température dans la gamme de 0°C à 200°C.

12. Formulation pharmaceutique comprenant un composé selon l'une quelconque des revendications 1 à 10, ou un sel pharmaceutiquement acceptable de celui-ci, en mélange avec des adjuvants, des diluants et/ou des supports pharmaceutiquement acceptables.

13. Composé selon l'une quelconque des revendications 1 à 10, ou un sel pharmaceutiquement acceptable de celui-ci, destiné à être utilisé comme médicament en thérapie.

14. Utilisation d'un composé selon l'une quelconque des revendications 1 à 10, dans la fabrication d'un médicament destiné au traitement et/ou à la prophylaxie de maladies cardiovasculaires.

15. Utilisation d'un composé selon l'une quelconque des revendications 1 à 10, dans la fabrication d'un médicament destiné au traitement et/ou à la prophylaxie de l'athérosclérose.

16. Utilisation d'un composé selon l'une quelconque des revendications 1 à 10, dans la fabrication d'un médicament destiné au traitement et/ou à la prophylaxie de l'hypercholestérolémie.

17. Utilisation d'un composé selon l'une quelconque des revendications 1 à 10, dans la fabrication d'un médicament destiné au traitement et/ou à la prophylaxie d'affections associées à un besoin d'améliorer le transport inverse du cholestérol.

18. Utilisation d'un composé selon l'une quelconque des revendications 1 à 10, dans la fabrication d'un médicament destiné au traitement et/ou à la prophylaxie d'affections associées à un besoin de diminuer l'absorption intestinale du cholestérol.

19. Utilisation d'un composé selon l'une quelconque des revendications 1 à 10, dans la fabrication d'un médicament destiné au traitement et/ou à la prophylaxie d'affections associées à un besoin d'augmenter les taux du cholestérol HDL.

20. Utilisation d'un composé selon l'une quelconque des revendications 1 à 10, dans la fabrication d'un médicament destiné au traitement et/ou à la prophylaxie d'affections associées à un besoin de diminuer les taux du cholestérol LDL.

21. Utilisation d'un composé selon l'une quelconque des revendications 1 à 10, dans la fabrication d'un médicament destiné au traitement et/ou à la prophylaxie d'affections inflammatoires.

22. Utilisation d'un composé selon l'une quelconque des revendications 1 à 10, dans la fabrication d'un médicament destiné au traitement et/ou à la prophylaxie de la maladie d'Alzheimer.

23. Utilisation d'un composé selon l'une quelconque des revendications 1 à 10, dans la fabrication d'un médicament destiné au traitement et/ou à la prophylaxie de l'artériosclérose.

24. Utilisation d'un composé selon l'une quelconque des revendications 1 à 10, dans la fabrication d'un médicament destiné au traitement et/ou à 1a prophylaxie du diabète de type 2.

25. Utilisation d'un composé selon l'une quelconque des revendications 1 à 10, dans la fabrication d'un médicament destiné au traitement et/ou à la prophylaxie des troubles lipidiques (dyslipidémie) qu'ils soient associés ou non à la résistance à l'insuline.

26. Formulation pharmaceutique destinée à être utilisée dans le traitement ou la prophylaxie d'affections associées à un besoin de moduler les récepteurs nucléaires des hormones LXR α et/ou β, comprenant un composé selon l'une quelconque des revendications 1 à 10 en tant que principe actif en mélange avec un adjuvant, un diluant ou un support pharmaceutiquement acceptable.

27. Composition pharmaceutique comprenant un composé selon l'une quelconque des revendications 1 à 10, combiné avec un autre agent thérapeutique qui est utile dans le traitement d'affections ou de troubles associés au développement et à l'évolution de l'athérosclérose tels que l'hypertension, les dyslipidémies, les hyperlipidémies, les hypercholestérolémies, le diabète de type 2, l'inflammation, l'obésité ainsi que des affections associées à un besoin d'améliorer le transport inverse du cholestérol et/ou de diminuer l'absorption intestinale du cholestérol.
